# EUROPEAN PATENT SPECIFICATION

(11) **EP 0 978 565 B1**
(45) Date of publication and mention of the grant of the patent: **10.10.2007**
(21) Application number: 99901926.8
(22) Date of filing: 29.01.1999
(51) Int. Cl.: C12N 15/62, C12P 21/02, C12P 21/06, A61K 38/28, C12N 1/21, C12N 5/10, C07K 14/605

(54) **PROCESS FOR PRODUCING PEPTIDE WITH THE USE OF ACCESSORY PEPTIDE**
VERFAHREN ZUR HERSTELLUNG EINES PEPTIDS MITTELS EINES HILFSPEPTIDS
PROCEDE DE PRODUCTION DE PEPTIDE AU MOYEN D'UN PEPTIDE ACCESSOIRE

(30) Priority: 30.01.1998 JP 3227298
(43) Date of publication of application: 09.02.2000
(73) Proprietor: Asubio Pharma Co., Ltd., Minato-ku Tokyo (JP)
(72) Inventor: OHSUYE, Kazuhiro, Ohta-shi, Gunma 373-0042 (JP); YABUTA, Masayuki, Tatebayashi-shi, Gunma 374-0038 (JP); SUZUKI, Yuji, Ashikaga-shi, Tochigi 326-0831 (JP)
(74) Representative: Stoner, Gerard Patrick
(86) International application number: PCT/JP1999/000406
(87) International publication number: WO 1999/038984

(56) References cited:
- JP-A- 5 328 992
- JP-A- 8 187 094
- JP-A- 9 296 000
- SUZUKI Y ET AL: "Effect of amino acid substitution at the P(3) and P(4) subsites of fusion proteins on kex2 protease activity." BIOTECHNOLOGY AND APPLIED BIOCHEMISTRY. ENGLAND AUG 2000, vol. 32 ( Pt 1), August 2000 (2000-08), pages 53-60, XP009007087 ISSN: 0885-4513
- MOTIN V L ET AL: "IMMUNIZATION WITH A PEPTIDE CORRESPONDING TO CHLAMYDIAL HEAT SHOCK PROTEIN 60 INCREASES THE HUMORAL IMMUNE RESPONSE IN C3H MICE TO A PEPTIDE REPRESENTING VARIABLE DOMAIN 4 OF THE MAJOR OUTER MEMBRANE PROTEIN OF CHLAMYDIA TRACHOMATIS" CLINICAL AND DIAGNOSTIC LABORATORY IMMUNOLOGY, AMERICAN SOCIETY FOR MICROBIOLOGY, US, vol. 6, no. 3, May 1999 (1999-05), pages 356-363, XP000881951 ISSN: 1071-412X

### SEQUENCE LISTING

<110> Suntory Limited
<120> Process for producing peptides using a helper peptide
<130> GPS/FP5805494
<140> EP 99901926.8
   <141> 1999-01-29
<150> PCT/JP99/00406
   <151> 1999-01-29
<150> JP 10-032272
   <151> 1998-01-30
<160> 24
<170> PatentIn Ver. 2.1
<210> 1
   <211> 4
   <212> PRT
   <213> Artificial Sequence
<220>
   <223> Amino acid sequence adjacent to a site cleaved by enterokinase
<400> 1
   Asp Asp Asp Lys
   1
<210> 2
   <211> 4
   <212> PRT
   <213> Artificial Sequence
<220>
   <223> Amino acid sequence adjacent to a site cleaved by blood coagulation Factor Xa
<400> 2
   Ile Glu Gly Arg
   1
<210> 3
   <211> 7
   <212> PRT
   <213> Artificial Sequence
<220>
   <223> Amino acid sequence containing a site cleaved by renin
<400> 3
   Pro Phe His Leu Leu Val Tyr
   1 5
<210> 4
   <211> 6
   <212> PRT
   <213> Artificial Sequence
<220>
   <223> Description of Artificial Sequence: Sequence cleavable at the C-terminal by entero-peptidase
<400> 4
   Val Asp Asp Asp Asp Lys
   1 5
<210> 5
   <211> 6
   <212> PRT
   <213> Artificial Sequence
<220>
   <223> Amino acid sequence of helper peptide
<400> 5
   Gly Cys His His His His
   1 5
<210> 6
   <211> 10
   <212> PRT
   <213> Artificial Sequence
<220>
   <223> Amino acid sequence containing a chemically cleaved site
<400> 6
   Pro Gly Gly Arg Pro Ser Arg His Lys Arg
   1 5 10
<210> 7
   <211> 10
   <212> PRT
   <213> Artificial Sequence
<220>
   <223> Amino acid sequence of helper peptide
<400> 7
   His Arg His Lys Arg Ser His His His His
   1 5 10
<210> 8
   <211> 5
   <212> PRT
   <213> Artificial Sequence
<220>
   <223> Amino acid sequence containing a site cleaved by Kex2 protease
<400> 8
   Ser Asp His Lys Arg
   1 5
<210> 9
   <211> 23
   <212> PRT
   <213> Artificial Sequence
<220>
   <223> Amino acid sequence of containing a position cleaved by OmpT
<400> 9
   Gln Met His Gly Tyr Asp Ala Glu Leu Arg Leu Tyr Arg Arg His His
   1 5 10 15
   Arg Trp Gly Arg Ser Gly Ser
   20
<210> 10
   <211> 20
   <212> PRT
   <213> Artificial Sequence
<220>
   <223> Amino acid sequence containing a position cleaved by OmpT
<400> 10
   Gln Met His Gly Tyr Asp Ala Glu Leu Arg Leu Tyr Arg Arg His His
   1 5 10 15
   Gly Ser Gly Ser
   20
<210> 11
   <211> 69
   <212> DNA
   <213> Artificial Sequence
<220>
   <221> CDS
   <222> (1)..(69)
<220>
   <223> Nucleotide sequence coding for an amino acid sequence containing a site cleaved by OmpT
<400> 11
   cag atg cat ggt tat gac gcg gag ctc cgg ctg tat cgc cgt cat cac    48
   Gln Met His Gly Tyr Asp Ala Glu Leu Arg Leu Tyr Arg Arg His His
    1               5                  10                  15
   cgg tgg ggt cgt tcc gga tcc                                         69
   Arg Trp Gly Arg Ser Gly Ser
               20
<210> 12
   <211> 23
   <212> PRT
   <213> Artificial Sequence
<220>
   <223> Amino acid sequence containing a site cleaved by OmpT
<400> 12
   Gln Met His Gly Tyr Asp Ala Glu Leu Arg Leu Tyr Arg Arg His His
    1               5                  10                  15
   Arg Trp Gly Arg Ser Gly Ser
               20
<210> 13
   <211> 47
   <212> DNA
   <213> Artificial Sequence
<220>
   <223> Nucleotide sequence coding for an amino acid sequence containing a site cleaved by OmpT
<400> 13
   tggttatgac gcggagctcc gcctgtatcg ccgtcatcac ggttccg 47
<210> 14
   <211> 55
   <212> DNA
   <213> Artificial Sequence
<220>
   <223> Nucleotide sequence coding for an amino acid sequence containing a site cleaved by OmpT
<400> 14
   gatccggaac cgtgatgacg gcgatacagg cggagctccg cgtcataacc atgca 55
<210> 15
   <211> 24
   <212> DNA
   <213> Artificial Sequence
<220>
   <223> Description of Artificial Sequence: Primer
<400> 15
   gactcagatc ttcctgaggc cgat 24
<210> 16
   <211> 36
   <212> DNA
   <213> Artificial Sequence
<220>
   <223> Description of Artificial Sequence: Primer
<400> 16
   aaaggtacct tccgcatgcc gcggatgtcg agaagg 36
<210> 17
   <211> 20
   <212> DNA
   <213> Artificial Sequence
<220>
   <223> Description of Artificial Sequence: Primer
<400> 17
   aggccaggaa ccgtaaaaag 20
<210> 18
   <211> 29
   <212> DNA
   <213> Artificial Sequence
<220>
   <223> Description of Artificial Sequence: Primer
<400> 18
   aaaatgcatc gcatcgtaac cgtgcatct 29
<210> 19
   <211> 627
   <212> DNA
   <213> Artificial Sequence
<220>
   <221> CDS
   <222> (82)..(546)
<220>
   <223> Nucleotide sequence coding for a fusion protein comprising GLP-1, helper peptide and beta -galactosidase protective peptide
<400> 19
<210> 20
   <211> 154
   <212> PRT
   <213> Artificial Sequence
<220>
   <223> Amino acid sequence of a fusion protein comprising GLP-1, helper peptide and beta-galactosidase protective peptide
<400> 20
   Met Thr Met Ile Thr Asp Ser Leu Ala Val Val Leu Gln Arg Lys Asp
   1 5 10 15
   Trp Asp Asn Pro Gly Val Thr Gln Leu Asn Arg Leu Ala Ala His Pro
   20 25 30
   Pro Phe Ala Ser Trp Arg Asn Ser Asp Asp Ala Arg Thr Asp Arg Pro
   35 40 45
   Ser Gln Gln Leu Arg Ser Leu Asn Gly Glu Trp Arg Phe Ala Trp Phe
   50 55 60
   Pro Ala Pro Glu Ala Val Pro Ala Ser Leu Leu Glu Ser Asp Leu Pro
   65 70 75 80
   Glu Ala Asp Thr Val Val Val Pro Ser Asn Trp Gln Met His Gly Tyr
   85 90 95
   Asp Ala Met His Gly Tyr Asp Ala Glu Leu Arg Leu Tyr Arg Arg His
   100 105 110
   His Gly Ser Gly Ser Pro Ser Arg His Pro Arg His Ala Glu Gly Thr
   115 120 125
   Phe Thr Ser Asp Val Ser Ser Tyr Leu Glu Gly Gln Ala Ala Lys Glu
   130 135 140
   Phe Ile Ala Trp Leu Val Lys Gly Arg Gly
   145 150
<210> 21
   <211> 187
   <212> PRT
   <213> Artificial Sequence
<220>
   <223> Amino acid sequence of a fusion protein comprising GLP-1, helper peptide and beta-galactosidase protective peptide
<400> 21
   Met Thr Met Ile Thr Asp Ser Leu Ala Val Val Leu Gln Arg Lys Asp
   1               5                   10                  15
   Trp Asp Asn Pro Gly Val Thr Gln Leu Asn Arg Leu Ala Ala His Pro
               20                  25                  30
   Pro Phe Ala Ser Trp Arg Asn Ser Asp Asp Ala Arg Thr Asp Arg Pro
           35                  40                  45
   Ser Gln Gln Leu Arg Ser Leu Asn Gly Glu Trp Arg Phe Ala Trp Phe
       50                  55                  60
   Pro Ala Pro Glu Ala Val Pro Ala Ser Leu Leu Glu Ser Asp Leu Pro
   65                  70                  75                  80
   Glu Ala Asp Thr Val Val Val Pro Ser Asn Trp Gln Met His Gly Tyr
                   85                  90                  95
   Asp Ala Pro Ile Tyr Thr Asn Val Thr Tyr Pro Ile Thr Val Asn Pro
               100                 105                 110
   Pro Phe Val Pro Thr Glu Pro His His His His His Gly Gly Arg Gln
           115                 120                 125
   Met His Gly Tyr Asp Ala Glu Leu Arg Leu Tyr Arg Arg His His Arg
   130 135 140
   Trp Gly Arg Ser Gly Ser Pro Ser Arg His Lys Arg His Ala Glu Gly
   145 150 155 160
   Thr Phe Thr Ser Asp Val Ser Ser Tyr Leu Glu Gly Gln Ala Ala Lys
   165 170 175
   Glu Phe Ile Ala Trp Leu Val Lys Gly Arg Gly
   180 185
<210> 22
   <211> 184
   <212> PRT
   <213> Artificial Sequence
<220>
   <223> Amino acid sequence of a fusion protein comprising GLP-1, helper peptide and beta-galactosidase protective peptide
<400> 22
   Met Thr Met Ile Thr Asp Ser Leu Ala Val Val Leu Gln Arg Lys Asp
   1 5 10 15
   Trp Asp Asn Pro Gly Val Thr Gln Leu Asn Arg Leu Ala Ala His Pro
   20 25 30
   Pro Phe Ala Ser Trp Arg Asn Ser Asp Asp Ala Arg Thr Asp Arg Pro
   35 40 45
   Ser Gln Gln Leu Arg Ser Leu Asn Gly Glu Trp Arg Phe Ala Trp Phe
   50 55 60
   Pro Ala Pro Glu Ala Val Pro Ala Ser Leu Leu Glu Ser Asp Leu Pro
   65 70 75 80
   Glu Ala Asp Thr Val Val Val Pro Ser Asn Trp Gln Met His Gly Tyr
   85 90 95
   Asp Ala Pro Ile Tyr Thr Asn Val Thr Tyr Pro Ile Thr Val Asn Pro
   100 105 110
   Pro Phe Val Pro Thr Glu Pro His His His His His Gly Gly Arg Gln
   115 120 125
   Met His Gly Tyr Asp Ala Glu Leu Arg Leu Tyr Arg Arg His His Gly
   130 135 140
   Ser Gly Ser Pro Ser Arg His Lys Arg His Ala Glu Gly Thr Phe Thr
   145 150 155 160
   Ser Asp Val Ser Ser Tyr Leu Glu Gly Gln Ala Ala Lys Glu Phe Ile
   165 170 175
   Ala Trp Leu Val Lys Gly Arg Gly
   180
<210> 23
   <211> 184
   <212> PRT
   <213> Artificial Sequence
<220>
   <223> Amino acid sequence of a fusion protein comprising GLP-1, helper peptide and beta-galactosidase protective peptide
<400> 23
   Met Thr Met Ile Thr Asp Ser Leu Ala Val Val Leu Gln Arg Lys Asp
   1 5 10 15
   Trp Asp Asn Pro Gly Val Thr Gln Leu Asn Arg Leu Ala Ala His Pro
   20 25 30
   Pro Phe Ala Ser Trp Arg Asn Ser Asp Asp Ala Arg Thr Asp Arg Pro
   35 40 45
   Ser Gln Gln Leu Arg Ser Leu Asn Gly Glu Trp Arg Phe Ala Trp Phe
   50 55 60
   Pro Ala Pro Glu Ala Val Pro Ala Ser Leu Leu Glu Ser Asp Leu Pro
   65 70 75 80
   Glu Ala Asp Thr Val Val Val Pro Ser Asn Trp Gln Met His Gly Tyr
   85 90 95
   Asp Ala Pro Ile Tyr Thr Asn Val Thr Tyr Pro Ile Thr Val Asn Pro
   100 105 110
   Pro Phe Val Pro Thr Glu Pro His His His His His Gly Gly Arg Gln
   115 120 125
   Met His Gly Tyr Asp Ala Glu Leu Arg Leu Tyr Arg Arg His His Gly
   130 135 140
   Ser Gly Ser Pro Ser Arg His Pro Arg His Ala Glu Gly Thr Phe Thr
   145 150 155 160
   Ser Asp Val Ser Ser Tyr Leu Glu Gly Gln Ala Ala Lys Glu Phe Ile
   165 170 175
   Ala Trp Leu Val Lys Gly Arg Gly
   180
<210> 24
   <211> 5
   <212> PRT
   <213> Artificial Sequence
<220>
   <223> Amino acid sequence containing a site cleaved by Kex2 Protease
<400> 24
   Ser Cys His Lys Arg
    1               5

## Description

### Technical Field

The present invention relates to a process for producing peptides using genetic enginerring technology.

### Background Art

Many biologically active peptides have been produced by genetic engineering technology using microorganisms, animal cells, and the like as hosts. As processes for producing a peptide of interest, there are known so-called direct expression method such as a process for extracellularly secreting a peptide, or a process for expressing a peptide from the N-terminal in the cell; or a process for expressing a fusion protein in which a protective peptide has been added to the N-terminal or the C-terminal of the peptide, or the like. Processes have been used wherein a peptide of interest is expressed either inside or outside of the cell, the expression product is then subjected to chemical or enzymatic cleavage or modification to produce the peptide of interest, which is then purified in a purification process to yield the peptide.

For the production of low molecular weight peptides, the fusion protein expression process mentioned above is generally used in order to avoid degradation of the protein by intracellular proteolytic enzymes. In this case, processes have been used wherein after intracellular expression of a fusion protein having a cleavage site region added thereto in between the peptide of interest and the protective peptide said cleavage site region being designed so as to permit cleavage by a chemical or enzymatic reaction, the peptide of interest is cleaved from the fusion protein by a chemical or enzymatic means, and the peptide is isolated and purified by a precipitation or a chromatographic process.

Furthermore, when the protein of interest is a protein of which a C-terminal has been amidated, such as calcitonin, a peptide having a glycine residue added to the C-terminal end of said peptide is expressed as a part of a fusion protein, the glycine-extended peptide of interest is cleaved from the fusion protein by a proteolytic enzyme, an amidating enzyme which is a modification enzyme is allowed to act on the peptide to produce an amidated peptide, and the amidated peptide of interest is obtained through purification processes.

However, when the production of peptides on an industrial scale is desired, various problems may arise as to the solubility and gelling of the peptide of interest in various conditions for cleaving and modification reactions, the concentration of a sample to be loaded to the column in the chromatographic process, the elution condition from the column, stability after elution, and the like. Most of the problems are caused by various physicochemical properties of the peptide of interest.

As the peptide of interest, for example, there can be mentioned human glucagon-like peptide-1 (referred to hereinafter as GLP-1; Bell GI et al., Nature, Vol. 304, pp. 368-371, 1983) and derivatives of GLP-1 (referred to hereinafter as GLP-1 derivatives) having an insulinotropic activity. GLP-1 is a peptide consisting of 37 amino acid residues derived from preproglucagon. Through processing of preproglucagon, GLP-1(7-37) in which 6 amino acids at the N-terminal of GLP-1 are deleted and GLP-1(7-36)NH₂ in which the C-terminal of GLP-1(7-36) has been modified to an amide form have been biosynthesized (Mojsow, S. et al., J. Clin. Invest. Vol. 79, pp. 616-619, 1987). Since these peptide hormones have an activity of promoting the secretion of insulin by acting on the beta cells in the pancreas, the possibility as candidates of a therapeutic agent for diabetics has been proposed based on their pharmacological effects in recent years (Gutniak M. K., et al., New England Medicine, Vol. 326, pp. 1316-1322, 1992; Nathan D. M., et al., Diabetes Care, Vol. 15, pp. 270-275, 1992).

As a process for producing the above peptide, a case may be contemplated in which the peptide is produced by a fusion protein expression method using E. coli etc. as a host in the conventional method mentioned above. In the case of GLP-1(7-37), for example, it is expressed as a fusion protein in which a protective peptide is added to the N-terminal or C-terminal end of GLP-1(7-37) through a cleavage site region for excising out GLP-1(7-37) from the fusion protein either chemically or enzymatically, and then GLP-1(7-37) can be excised out from the fusion protein either chemically or enzymatically to produce GLP-1(7-37). And, GLP-1(7-36)NH₂ can be produced by adding a step of modification reaction to the above process. Thus, GLP-1(7-37) is expressed as a fusion protein as mentioned above as a substrate for an amidation enzyme for the amidation modification reaction wherein GLP-1(7-37) can be considered as an intermediate peptide for the production of GLP-1(7-36)NH₂. GLP-1(7-37) is excised from the fusion protein either chemically or enzymatically, and then the GLP-1(7-37) thus obtained is subjected to an amidation modification reaction using an amidation enzyme to produce the desired GLP-1(7-36)NH₂.

However, even when the above mentioned processes are used to produce a peptide of interest, a GLP-1 derivative having an insulinotropic activity, undesirable problems on production processes arise. Therefore, production processes that permit inexpensive supply on an industrial level have not been established yet, and thus there is a strong need for the establishment of such methods.

For example, as a process for producing GLP-1(7-37) as a peptide of interest a fusion protein comprising a protective peptide and GLP-1(7-37) is expressed in an conventional process, as mentioned above, and GLP-1(7-37) can be produced by excising it directly from said protein. Thus, the purification steps of the production process can use (1) enzymatic excision step of GLP-1(7-37) from the fusion protein and (2) a chromatographic step. However, since GLP-1(7-37) can easily gel or aggregate during purification, problems on production processes that are derived from physicochemical properties, such as markedly reduced recovery and incapability of resin regeneration, may sometimes arise. Once it has been gelled, though it can be solubilized at pH 10 or higher and can be purified, problems such as undesirable modifications and conformational changes may appear (Senderoff RI, et al., J. Pharm. Sci., Vol. 87, pp. 183-189, 1998).

Furthermore, when the peptide of interest is an amidated peptide GLP-1(7-36)NH₂, problems may arise as to the amidation reaction. Thus, optimal pH of enzymatic amidation reactions is approximately from weak acid to neutral, whereas the theoretical isoelectric points of GLP-1(7-37) and GLP-1(7-36)NH₂ are pI = 5.5 and pI = 7.5, respectively. Therefore, when an enzymatic conversion to GLP-1(7-37) is carried out at the optimal pH of an amidation enzyme that is near the isoelectric point of the substrate GLP-1(7-37), it is believed that GLP-1(7-37) tends to form an isoelectric precipitation.

Furthermore, since GLP-1(7-36)NH₂ that was formed by the precipitated GLP-1(7-37) also coprecipitates, enzyme reactions may not fully proceed causing problems in production processes. Furthermore, since GLP-1(7-36) is also a peptide that tends to aggregate in handling during the column step and to gel in the column, problems as to purification may also arise. Thus, it is expected that GLP-1 derivatives also have the above-mentioned problems, in production processes, that are derived from physicochemical properties.

In the production of GLP-1(7-37), GLP-1(7-36)NH₂ and GLP-1 derivatives on an industrial scale, the above-mentioned problems can cause problems on industrial production derived from the physicochemical properties of the peptide of interest itself, causing serious problems in terms of yield, process control, and even production cost.

### Disclosure of the Invention

It is an object of the present invention to provide a process for producing a peptide of interest in an efficient manner by relieving the problems derived from the physicochemical properties of the peptide of interest itself (for example, the problem of low solubility and gelling in the chemical and enzymatic reaction or the purification step in the production processes of the peptide of interest), when an efficient production of said peptide of interest on an industrial scale using genetic engineering technology is contemplated.

The term "the peptide of interest of the present invention" as used herein means not only the peptide finally obtained but also the production intermediate peptides that are required in the production process thereof.

In order to avoid the above-mentioned problems, the inventors of the present invention have found a process for producing a peptide of interest in an efficient manner by relieving the problems of the peptide of interest by adding a helper peptide to the peptide of interest. Thus, the present invention provides a process as defined in the claims.

### Brief Description of Drawings

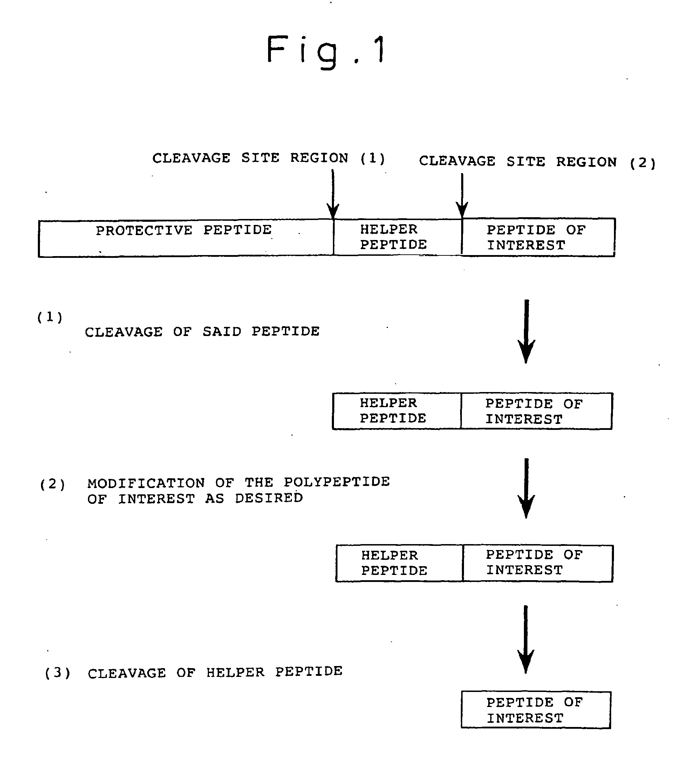

Figure 1 outlines a process for producing the peptide of interest using a helper peptide.
Figure 2 is a drawing that shows a process for constructing pG117S4HR6GLP-1.
Figure 3 is a drawing that shows a process for constructing pGP117S4HompRHKR.
Figure 4 is a drawing that shows a process for constructing pGP117S4HompRHPR.
Figure 5 is a drawing that shows a process for constructing pGP97ompPR.
Figure 6 is a drawing that shows the oligonucleotides and the primers used for constructing pGP97ompPR.
Figure 7 is a drawing that shows an amino acid sequence of a fusion protein (GP97ompPR) encoded by pGP97ompPR. The underlined part indicates the amino acid sequence derived from GLP-1(7-37), and the double-underlined part indicates the sequence of a helper peptide. ↓ indicates the cleavage site by the E. coli OmpT protease, and the arrow after the double-underline indicates the cleavage site by Kex2 protease.
Figure 8 is a drawing that shows the nucleotide sequence of DNA of a fusion protein (GP97ompPR) encoded by pG97ompPR, wherein the region from A of the nucleotide number 1 to T of the nucleotide number 462 encodes the fusion protein of GLP-1(7-37). lac PO represents the promoter/operator region of the E. coli lactose operon.
Figure 9 is a photograph of an electrophoresis that shows the culturing of a recombinant E. coli cells and an expression of a fusion protein (GP97ompPR). In the figure are shown the results of the SDS-polyacrylamide gel electrophoresis of the samples at sampling times a through e. The arrow in the figure indicates the band of the fusion protein.
Figure 10 is a drawing that shows an analysis of the cleavage of a fusion protein (GP97ompPR) with the E. coli OmpT protease present in the inclusion bodies.
Figure 11 is a drawing that shows an amino acid sequence of a fusion protein encoded by pG117S4HR6GLP-1. In the figure, the underlined part indicates an amino acid sequence derived from GLP-1(7-37), and the double-underlined part indicates an amino acid sequence derived from a helper peptide.
Figure 12 is a drawing that shows an amino acid sequence of a fusion protein encoded by pGP117S4HompRHKR. In the figure, the underlined part indicates an amino acid sequence derived from GLP-1(7-37), and the double-underlined part indicates an amino acid sequence derived from a helper peptide.
Figure 13 is a drawing that shows an amino acid sequence of a fusion protein encoded by pGP117S4HompRHPR. In the figure, the underlined part indicates an amino acid sequence derived from GLP-1(7-37), and the double-underlined part indicates an amino acid sequence derived from a helper peptide.
Figure 14 is a drawing that shows an elution pattern of RHHGP[G] from the SP Sepharose Big beads, wherein the starting position of elution is shown by ↓and the pooled fractions are shown in the figure. Absorbance was measured at 280 nm.
Figure 15 is a drawing that shows an analytical pattern at each purification step in the cleavage process of GLP-1(7-37) from RHHGP[G] by Kex2 protease. In the figure, A indicates a reverse-phase pool before cleavage with Kex2 protease, B after cleavage with the Kex2 protease, and C after PorosR2, and 1 indicates RHHGP[G] and 2 indicates GLP-1(7-37).
Figure 16 is a drawing that shows the pH dependency of the amidation reaction of RHHGP[G].
Figure 17 is a drawing that shows a time course of the conversion of RHHGP[G] to RHHGP-1 in an amidation reaction as measured by an ion exchange HPLC, wherein 1 indicates RHHGP[G] and 2 indicates RHHGP-1. Absorbance was measured at 280 nm.
   The analytical condition was as follows:
   Column: Poros S/H, 4.6 mm I.D. x 50 mm
   Flow rate: 1.6 ml/min
   Solution A: 30 mM BR buffer, pH 6.0
   Solution B: 30 mM BR buffer, pH 9.0
   Equilibration: solution A
   Elution: solution B 0% → 100%, linear pH gradient.
Figure 18 is a drawing that shows the pH dependency of a Kex2 protease processing reaction with RHHGP-1 as a substrate.
Figure 19 is a drawing that shows an elution pattern and a pH gradient created, in the purification of GLP-1(7-36)NH₂ by Macroprep High-S. Absorbance was measured at 280 nm.
Figure 20 is a drawing that shows the removal of impurities with Macroprep High-S, wherein A indicates the sample loaded to the column, B indicates an analytical HPLC pattern. 1 indicates GLP-1(7-37) and 2 indicates GLP-1(7-36) NH₂. Absorbance was measured at 280 nm.
Figure 21 is a drawing that collectively shows the analytical HPLC patterns of samples in each purification process in the production of GLP-1(7-36)NH₂ through the first cleavage with OmpT and the second cleavage with Kex2 protease. A indicates a reverse-phase HPLC pattern after OmpT, B after SP Sepharose, C after Kex2 reaction, D after Macroprep High-S, and E after Poros R2. 1 indicates GP97ompPR, 2 indicates the protective peptide, 3 indicates RHHGP[G], 4 indicates RHHGP-1, and 5 indicates GLP-1(7-36) NH₂. Absorbance was measured at 280 nm.
   The analytical condition was as follows:
   Column: YMC Protein RP, 4.6 mm I.D. x 50 mm
   Flow rate: 1.0 ml/min
   Solution A: 0.1% TFA / 10% acetonitrile
   Solution B: 0.1% TFA / 60% acetonitrile
   Equilibration: solution A
   Elution: solution B 44% → 74% /12 min.
Figure 22 is a drawing that shows the pH dependency of the solubility of RHHGP[G], RHHGP-1, GLP-1(7-37), and GLP-1(7-36)NH₂.
Figure 23 is a drawing that shows a suppressing effect on the aggregation of RHHGP[G], RHHGP-1, and GLP-1(7-36)NH₂ by Tween 80, wherein A indicates the suppression of aggregation of RHHGP[G] and RHHGP-1 by Tween 80, and B indicates the suppression of aggregation of GLP-1(7-36)NH₂ by Tween 80.
Figure 24 is a drawing that shows a suppressing effect on the aggregation of GLP-1(7-36)NH₂ by NaCl and temperature, wherein C indicates the suppression of aggregation of GLP-1[G] by NacT and D indicates the suppression of aggregation of GLP-1(7-36)NH₂ by temperature.

### Embodiments for Carrying Out the Invention

The helper peptide according to the present invention is a peptide that is used to circumvent the problems on industrial production derived from physicochemical properties of the peptide of interest itself. Of the problems on industrial production derived from physicochemical properties of the peptide of interest itself, special attention is paid to the problem in chemical or enzymatic reactions and purification during the production process of said peptide, such as problems of solubility and gelling of the peptide of interest under various conditions of cleavage and modification reactions, the problem of sample concentration to be loaded on the column during the column chromatographic process, the problem of the elution conditions from the column and the stability after elution, and the like.

The helper peptide of the present invention may be prepared as appropriate depending on the physicochemical properties of the peptide of interest. For example, when the isoelectric point of the peptide of interest is neutral to weak acid and the optimum pH during the production process is also neutral to weak acid and thereby the solubility of the peptide of interest under such pH is too low, then the helper peptide is preferably designed so that the isoelectric point (pI) of the peptide of interest that has a helper peptide added thereto is 8 to 12 and more preferably 9 to 11. The helper peptide of the present invention may be added to either the N-terminal or C-terminal of the peptide of interest. The dimension (length) of a helper peptide is preferably 5 to 50 amino acid residues, and more preferably from 5 to not greater than 30 amino acid residues, but it contains at least one basic amino acid or acidic amino acid.

In addition to the above-mentioned GLP-1 derivatives, the peptides of interest that can be produced according to the present invention include peptides comprising not greater than 200 amino acid residues. Examples of such peptides include adrenocorticotropic hormone, adrenomedullin, amylin, angiotensin I, angiotensin II, angiotensin III, A-type natriuretic peptide, B-type natriuretic peptide, bradykinin, big gastrin, calcitonin, calcitonin gene related peptide, cholecystokinin, corticotropin releasing factor, cortistatin, C-type natriuretic peptide, defesin 1, delta sleep-inducing peptide, dynorphin, elafin, α-endorphin, β-endorphin, γ-endorphin, endothelin-1, endothelin-2, endothelin-3, big endothelin-1, big endothelin-2, big endothelin-3, enkephalin, galanin, big gastrin, gastrin, gastric inhibitory polypeptide (GIP), gastrin releasing peptide, glucagon, glucagon-like peptide-2, growth hormone releasing factor, growth hormone, guanylin, uroguanylin, histatin 5, insulin, joining peptide, luteinizing hormone releasing hormone, melanocyte stimulating hormone, midkine, motilin, neurokinin A, neurokinin B, neuromedin B, neuromedin C, neuropeptide Y, neurotensin, oxytocin, proadrenomedullin N-terminal 20 peptide, cromogranin A, parathyroid hormone, PTH related peptide, peptide histidine-methionine-27, pituitary adenylate cyclase activating polypeptide 38, platelet factor-4, peptide T, secretin, serum thymic factor, somatostatin, substance P, thyrotropin releasing hormone, urocortin, vasoactive intestinal peptide, vasopressin, and the derivatives thereof, etc.

As the GLP-1 derivatives, in addition to the above-mentioned GLP-1(7-37) and GLP-1(7-36)NH₂, there can be mentioned peptides having an insulinotropic activity in which amino acid residues have been substituted for, added to, and/or deleted from the peptide comprising 37 amino acid residues of GLP-1, peptides having an insulinotropic activity in which amino acids of said peptide have been further modified (for example an amidated form), and peptides having an insulinotropic activity that are obtained from the combinations thereof.

Furthermore, as the GLP-1 derivatives that are suitably produced according to the method of the present invention, those GLP-1 derivatives that have isoelectric points from 4.5 to 9.0 are preferred. More preferably they are GLP-1 derivatives having isoelectric points from 5.5 to 7.5.

As the GLP-1 derivatives, in addition to those mentioned above, the following can be mentioned:
GLP-1(7-34), GLP-1(7-35), GLP-1(7-36), GLP-1(7-34)NH₂, GLP-1(7-35)NH₂, and GLP-1(7-37)NH₂;
GLP-1(7-37)-Arg, GLP-1(7-37)-Arg-Arg, GLP-1(7-37)-Lys, GLP-1(7-37)-Lys-Lys, GLP-1(7-37)-Lys-Arg, and GLP-1(7-37)-Arg-Lys, and the C-terminal-amidated forms thereof;
GLP-1(7-37) and GLP-1(7-36)NH₂ in which the amino acid at position 8, Ala, of GLP-1 has been substituted with Thr, Gly or Ser;
GLP-1(7-37) and GLP-1(7-36)NH₂ in which the amino acid at position 26, Lys, of GLP-1 has been substituted with Arg;
GLP-1(7-37) and GLP-1(7-36)NH₂ in which the amino acid at position 34, Lys, of GLP-1 has been substituted with Arg;
GLP-1(7-37) and GLP-1(7-36)NH₂ in which the amino acid at position 36, Arg, of GLP-1 has been substituted with Lys;
GLP-1(7-37) and GLP-1(7-36)NH₂ in which the amino acid at position 8, Ala, of GLP-1 has been substituted with Thr, Gly or Ser, and the amino acid at position 26, Lys, has been substituted with Arg;
GLP-1(7-37) and GLP-1(7-36)NH₂ in which the amino acid at position 8, Ala, of GLP-1 has been substituted with Thr, Gly or Ser, and the amino acid at position 34, Lys, has been substituted with Arg;
GLP-1(7-37) and GLP-1(7-36)NH₂ in which the amino acid at position 8, Ala, of GLP-1 has been substituted with Thr, Gly or Ser, and the amino acid at position 36, Arg, has been substituted with Lys;
GLP-1(7-37) and GLP-1(7-36)NH₂ in which the amino acid at position 26, Lys, of GLP-1 has been substituted with Arg, and the amino acid at position 34, Lys, has been substituted with Arg;
GLP-1(7-37) and GLP-1(7-36)NH₂ in which the amino acid at position 26, Lys, of GLP-1 has been substituted with Arg, and the amino acid at position 36, Arg, has been substituted with Lys;
GLP-1(7-37) and GLP-1(7-36)NH₂ in which the amino acid at position 34, Lys, of GLP-1 has been substituted with Arg, and the amino acid at position 36, Arg, has been substituted with Lys;
GLP-1(7-37) and GLP-1(7-36)NH₂ in which the amino acid at position 8, Ala, of GLP-1 has been substituted with Thr, Gly or Ser, the amino acid at position 26, Lys, of GLP-1 has been substituted with Arg, and the amino acid at position 34, Lys, has been substituted with Arg;
GLP-1(7-37) and GLP-1(7-36)NH₂ in which the amino acid at position 8, Ala, of GLP-1 has been substituted with Thr, Gly or Ser, the amino acid at position 26, Lys, of GLP-1 has been substituted with Arg, and the amino acid at position 36, Arg, has been substituted with Lys;
GLP-1(7-37) and GLP-1(7-36)NH₂ in which the amino acid at position 8, Ala, of GLP-1 has been substituted with Thr, Gly or Ser, the amino acid at position 34, Lys, of GLP-1 has been substituted with Arg, and the amino acid at position 36, Arg, has been substituted with Lys;
GLP-1(7-37) and GLP-1(7-36)NH₂ in which the amino acid at position 26, Lys, of GLP-1 has been substituted with Arg, the amino acid at position 34, Lys, of GLP-1 has been substituted with Arg, and the amino acid at position 36, Arg, has been substituted with Lys; and
GLP-1(7-37) and GLP-1(7-36)NH₂ in which the amino acid at position 8, Ala, of GLP-1 has been substituted with Thr, Gly or Ser, the amino acid at position 26, Lys, of GLP-1 has been substituted with Arg, and the amino acid at position 36, Arg, has been substituted with Lys.

In the case of GLP-1(7-37) as an example of the peptide of interest, a helper peptide having basic amino acids can be added to GLP-1(7-37) and then can be used for the production of GLP-1(7-37) in order to relieve the problems on purification processes such as the gelling and the solubility of GLP-1(7-37). Thus, by adding the helper peptide to GLP-1(7-37), the isoelectric point (pI = 5.5) of GLP-1(7-37) can be shifted to the alkali side and thereby the hydrophilicity can be increased so as to avoid the problems on purification processes such as aggregation (gelling) in the column. Furthermore, by adding said helper peptide to GLP-1(7-37), it has become possible to isolate a peptide comprising the helper peptide and GLP-1(7-37) at very high purity and high yields in the first chromatographic process, which results in enhanced recovery of GLP-1(7-37), indicating that the use of a helper peptide is very useful in this process.

In the case of GLP-1(7-36)NH₂ as an example of the peptide of interest, it is necessary to first obtain an intermediate product (said peptide is specifically GLP-1(7-37)) as the peptide of interest since GLP-1(7-36)NH₂ is an amidated peptide. Thus, a helper peptide having a basic amino acid can be added to GLP-1(7-37), which can be used for the production of GLP-1(7-36)NH₂. By adding a helper peptide having a basic amino acid, the isoelectric point of GLP-1(7-37) can be shifted to the alkali side and thereby precipitate formation can be suppressed due to increased solubility of the peptide having a helper peptide added to GLP-1(7-37) at the pH of the amidation enzymatic reaction liquid at a subsequent amidation modification reaction and thereby the recovery and the yield can be increased. By adding a hydrophilic helper peptide containing a basic amino acid, the solubility of the peptide of interest can also be increased, and furthermore the aggregation of GLP-1(7-37) as the substrate in the amidation modification reaction and GLP-1(7-36)NH₂ as a product can be avoided, thereby indicating that the addition is very useful in the purification process after the amidation enzymatic reaction.

In any of the above cases, the peptide having a helper peptide added to GLP-1(7-37) has preferably an isoelectric point of 8 to 12. By subjecting said peptide to the action of a cation ion exchange resin, said peptide can be obtained at high yields (98% or greater).

In order to obtain the peptide of interest from the peptide that has a helper peptide added thereto, a cleavage site region is introduced that permits chemical or enzymatic cleavage in between the helper peptide and the peptide of interest. For the cleavage site region as well, a cleavage site region having a high cleavage efficiency is designed depending on the physicochemical properties owned by the peptide of interest. As the enzymatic and chemical cleavage methods, those described in Methods in ENZYMOLOGY, Vol. 185, Gene Expression Technology (David V. Goeddel ed., published by ACADEMIC PRESS, INC.) can also be used.

As the chemical cleavage method, there can be mentioned a method in which the C-terminal end of methionine is cleaved with cyanogen bromide (D. V. Goeddel et al., Proc. Natl. Acad. Sci. U.S.A., Vol. 76, pp. 106-110, 1979), a method in which the -Asp-Pro-sequence is cleaved with formic acid (Biochem. Biophys. Res. Commun., Vol. 40, pp. 1173, 1970), a method in which -Asn-Gly- sequence is cleaved with hydroxylamine, a method in which the C-terminal end of trypsin is cleaved with BNPS-skatole or N-chlorosuccinimide, and the like. When methionine is not included in such an amino acid sequence of the peptide of interest, methionine is introduced at the end of the cleavage site region adjacent to the peptide of interest, and chemical cleavage with cyanogen bromide at the cleavage site region can be carried out.

As an enzymatic cleavage method, it is required to design a cleavage site region that can be specifically recognized as a substrate by an enzyme used for the cleavage treatment. Examples of such methods include the cleavage of: the -X-Gly- sequence of the X-Gly or the Pro-X-Gly-Pro sequence with collagenase (Proc. Natl. Acad. Sci. U.S.A., Vol. 81, pp. 4692-4696, 1984); the C-terminal end of Lys of the -Asp-Asp-Asp-Lys- sequence (SEQ ID NO: 1) with enterokinase; the C-terminal end of Arg of -Ile-Glu-Gly-Arg- sequence (SEQ ID NO: 2) with blood coagulation factor Xa (Japanese Unexamined Patent Publication (Kokai) No. 61(1986)-135591); the C-terminal end of Arg of the -Gly-Pro-Arg- sequence with thrombin (Japanese Unexamined Patent Publication (Kokai) No. 62(1987)-135500); the C-terminal end of -Arg- with trypsin or clostripain; the C-terminal end of Arg or Lys with endoprotease Arg-C (Nature, Vol. 285, pp. 456-461, 1980); the C-terminal end of the Lys-Arg, Arg-Arg, or Pro-Arg sequence with Saccharomyces cereviceae Kex2 protease and derivatives thereof (Biochem. Biophys. Res. Commun., Vol. 144, pp. 807-814, 1987, Japanese Unexamined Patent Publication (Kokai) No. 1(1989)-199578); the C-terminal end of Lys with lysyl endopeptidase or endopeptidase Lys-C (Japanese Unexamined Patent Publication (Kokai) No. 61(1986)-275222); the C-terminal end of Asp or Glu with Staphylococcus aureus (S. aureus) V8 protease (Proc. Natl. Acad. Sci. U.S.A., Vol. 69, pp. 3506-3509, 1972); the C-terminal end of the -Phe-Arg-sequence with kallikrein (Japanese Unexamined Patent Publication (Kokai) No. 62(1987)-248489); Leu-Leu of the -Pro-Phe-His-Leu-Leu-Vla-Tyr- sequence (SEQ ID NO: 3) with renin (Japanese Unexamined Patent Publication (Kokai) No. 60(1985)-262595); the C-terminal end of the -Glu-Gly-Arg- sequence with urokinase (Japanese Unexamined Patent Publication (Kokai) No. 2(1990)-100685); the C-terminal end of the Val-Asp-Asp-Asp-Asp-Lys sequence (SEQ ID NO: 4) with entero-peptidase (Biotechnology, Vol. 6, pp. 1204-1210, 1988); the C-terminal end of poly-Gly with lysostaphin (Japanese Unexamined Patent Publication (Kokaki) 1(1989)-160496); the C-terminal end of Lys-Arg, Arg-Arg, or Pro-Arg, etc. with Kluverromyces lactis) (Japanese Unexamined Patent Publication (Kokai) No. 1(1989)-124390), and the like.

In the Examples of the present invention, for example, an amino acid sequence (the Lys-Arg, Arg-Arg or Pro-Arg sequence) that can be recognized by Kex2 protease was introduced into the cleavage site region and then the peptide of interest was cleaved with the enzyme.

Depending on the substrate specificity of an enzyme used in the cleavage treatment and the amino acid sequence of the peptide of interest, therefore, it is preferred that one or more of methionine, tryptophan, proline, glycine, cysteine, arginine, lysine, aspartic acid, and glutamic acid is present in the amino acid sequence of the cleavage site region.

When modification is required to obtain a final peptide of interest (for example, an amidated peptide), a modification reaction (for example, an amidating modification reaction with an amidation enzyme) is carried out before or after excising said peptide of interest from the peptide of interest having a helper peptide added thereto (in this case the final peptide of interest is a production intermediate peptide). Furthermore, efficient modification reaction is desired, a modification reaction (for example, an amidation modification reaction) is performed on the peptide of interest having a helper peptide added thereto before excising the peptide of interest to obtain the final peptide to which a helper peptide has been added, and then the cleavage site region in between the helper peptide and the final peptide of interest is cleaved to obtain the final peptide of interest.

The peptide of interest is produced by further adding a protective peptide and expressing it as has been conducted in the conventional fusion protein method. Thus, it is possible to produce the peptide of interest at a high expression level within the host cell as a fusion protein further having a protective peptide added to the peptide of interest having a helper peptide added thereto (the protective peptide may be added to any of the N-terminal or C-terminal of the peptide of interest having a helper peptide added thereto).

The protective peptide for use in the production process of the present invention is not specifically limited and the conventionally used peptides can be used as appropriate with modifications. For example, in Japanese Unexamined Patent Publication (Kokai) No. 54(1979)-145289, fragments of the amino acid sequences from β-galactosidase derived from E. coli can be used as a protective peptide. The amino acid sequences of said enzyme are known to a person skilled in the art, and peptide fragments derived from β-galactosidase have been widely used as the protective peptide in the fusion protein method by persons skilled in the art. In the production processes of the present invention, peptide fragments that were obtained by appropriate modification of the amino acid sequences of β-galactosidase can be used as the protective peptide by taking into account the characteristics of the peptide of interest having a helper peptide added thereto. It is also possible to chemically synthesize a DNA base sequence encoding the amino acid sequence of the protective peptide.

It is preferred to devise and obtain different isoelectric points for each of the protective peptide, the peptide of interest having a helper peptide added thereto constituting said fusion protein, and said fusion protein, in order to enhance the fragment resolving ability during the chromatographic processes after the cleavage treatment reaction. The same is also true for the helper peptide and the peptide of interest.

It is also necessary to provide a cleavage site region in between the peptide of interest having a helper peptide added thereto and the protective peptide, but it is possible to provide a cleavage site region having a preferable high cleavage efficiency as appropriate according to the above-mentioned strategy. However, a multi-step method of cleaving the fusion protein is required at each site since a plurality of cleavage site regions have been provided. The first cleavage treatment is carried out in the cleavage site region in between the peptide of interest having a helper peptide added thereto and the protective peptide, and then a cleavage treatment is carried out in the cleavage site region in between the helper peptide and the peptide of interest thereby to obtain the peptide of interest.

In order to confirm the wide applicability of the above production process, processes were examined, in the chemical or enzymatic cleavage of each cleavage site region and it was confirmed that any method of cleavage treatment can be carried out. Representative examples of the site-specific cleavage method of peptide at such cleavage site regions include:
(1) a method in which the absence of cysteine residues in the amino acid sequences of the protective peptide and the peptide of interest is utilized, and cysteine is introduced in between the peptide of interest having a helper peptide added thereto and the protective peptide, which is then cyanized, subjected to an alkali treatment, and the fusion protein is specifically cleaved at the site;
(2) a method in which cleavage at each cleavage site region is carried out with the same enzyme, but by using different amino acid sequences at the enzyme recognition sites, the reaction condition for one cleavage site region is designed so as not to permit cleavage at another cleavage site region; and
(3) a method in which cleavage at each cleavage site region is carried out with the same enzyme, but by modifying amino acids in the cleavage site region (cleavage site region 2) present in between the helper peptide and the peptide of interest, the reaction condition for cleavage of the other cleavage site region (cleavage site region 1) present in between the peptide of interest having a helper peptide added thereto and the protective peptide is designed so as not to cause cleavage at cleavage site region 2, and after cleavage at cleavage site region 1, the peptide of interest having a helper peptide added thereto is purified, and then the modified amino acid is modified again so that cleavage site region 2 can be made possible to be cleaved with the above enzyme.

Host cells that can be used in the present invention are not specifically limited and the conventionally used prokaryotic cells or eukaryotic cells, for example microorganism cells such as E. coli, yeasts or animal cells that can suitably express, using an expression vector having a nucleotide sequence encoding, a peptide of interest having a helper peptide added thereto can be chosen as appropriate and used. Furthermore, other elements required for high expression such as a promoter, a terminator, and splice sites are also chosen from the conventionally used ones and used as appropriate.

In the production process of the peptide of interest of the present invention, the following describes a case wherein the peptide of interest is GLP-1(7-37) and GLP-1(7-36)NH₂.

The fusion protein (referred to hereinafter as GP97ompPR) encoded by the GLP-1(7-37) expression vector (referred to hereinafter as pGP97ompPR) is a fusion protein that has a peptide (referred to hereinafter as RHHGP[G]) having a helper peptide added thereto, said helper peptide containing a basic peptide region in the N-terminal end of GLP-1(7-37), and that has an E. coli β-galactosidase derivative (β-gal97S) added to the N-terminal end of RHHGP[G] as a protective peptide. A cleavage site region has been introduced in between the protective peptide and RHHGP[G] and between the helper peptide of RHHGP[G] and GLP-1(7-37). Each of the regions has amino acid sequences responsible for substrate specificity so that one cleavage site region may be cleaved with an endogenous OmpT protease derived from E. coli, and the other cleavage site region may be cleaved with Kex2 protease (Japanese Patent No. 2643968, and Japanese Unexamined Patent Publication (Kokai) 10(1998)-229884, and the like).

For GP97ompPR, the isoelectric point of each of β-gal97S or RHHGP[G] and GP97ompPR has been designed to be different from each other, in order to enhance the fragment resolving ability in the chromatographic process after the cleavage treatment reaction. For example, in Examples described below, the isoelectric point of GP97ompPR has been designed to be 5.95, that of β-gal97S to be 4.60, and that of RHHGP[G] to be 10.09.

Then, E. coli (W3110/pG97ompPR) transformed with pGP97ompPR was cultured to express GP97ompPR. GP97ompPR was highly expressed as an insoluble protein in the cells and was accumulated in the inclusion bodies. The final cell density reached to 180 at OD660 nm.

After disrupting the cells, GP97ompPR was solubilized with urea, and the cleavage site region in between the protective peptide β-gal97S and the peptide of interest RHHGP[G] having a helper peptide added thereto in GP97ompPR was cleaved with the endogenous OmpT protease existing in the inclusion body. OmpT protease specifically cleaved said cleavage site region with a cleavage efficiency of 85%.

Next, in order to separate β-gal97S and RHHGP[G] and to remove urea, a cation exchange chromatography was carried out. Since the isoelectric points of non-cleaved GP97ompPR (pI = 5.95) and that of β-gal97S (pI = 4.60) were both in the acid side they do not adsorb to the column, and RHHGP[G] (pI = 10.09) was adsorbed and eluted. Through this one step of column treatment alone, RHHGP[G] at a purity of 99% was obtained. The fact that the first stage of column procedures during the production process provides RHHGP[G] at such a high purity and high yield is very useful from the viewpoint of industrial large-scale production.

The cleavage site region in between the helper peptide and the peptide of interest present in RHHGP[G] (1.0 g) was cleaved using Kex2 protease. In the reaction condition described in Examples below, the reaction proceeded at a cleavage efficiency of 95%. The recovery was 90%.

Subsequently, a reverse-phase chromatography was carried out to further purify GLP-1(7-37). The recovery of this process was 80%, the final yield of the overall processes was about 64% and 0.72 g of GLP-1(7-37) having a purity of 98% was obtained. Since the amount of the culture broth used for purification was about 0.36 liter, this means that about 2.0 g was obtained per liter of the culture broth. The recovery and the yield are both very high and thereby have proved the usefulness of the production method of the present invention that employs a helper peptide. Thus, the production process of the present invention enables full realization of the production of the peptide of interest on an industrial scale.

Concerning the production of GLP-1(7-37) using the production process of the present invention, the yield at each step is shown in Table 2-B below.

As can be seen from Table 2-B, it was demonstrated that the yield at each step is very high and the finally recovery of 64% is very high.

Furthermore, when the peptide of interest is GLP-1(7-36)NH₂, an amidation modification reaction is required since the peptide is an amidated peptide. The peptide can be obtained as follows:
RHHGP[G] obtained as mentioned above was subjected to an amidation modification reaction using an amidation enzyme (B. B. R. C., Vol. 150, pp. 1275-1281, 1988, EP299790A, and the like). Under the reaction condition in Examples described below, no aggregation or gelling of RHHGP[G] as the enzyme substrate and the reaction product, amidated GLP-1(7-36)NH₂ (referred to hereinafter as RHHGP-1) having a helper peptide added thereto occurs, and RHHGP-1 could be produced at a high reaction rate of 98% (a recovery of 95%). These results demonstrated the usefulness of the helper peptide in the amidation enzymatic reaction conducted with a substrate of RHHGP[G].

After the amidation modification reaction, the cleavage site region in between the helper peptide and the peptide of interest present in RHHGP-1 was cleaved using Kex2 protease. Under the reaction condition in Examples described below, the reaction proceeded at a cleavage efficiency of 95% (a recovery of 90%) or greater.

In order to further purify GLP-1(7-36)NH₂, a cation exchange chromatography and then a hydrophobic chromatography were carried out. The final yield of the overall process was about 50% and 13.5 g of GLP-1(7-36)NH₂ with a purity of 98% was obtained. Since the amount of the culture broth used for purification was about 8 liters, this means that about 1.68 g was obtained per liter of the culture broth. The recovery and the yield are both very high and thereby it was demonstrated that the production method of the present invention that employs a helper peptide is very useful. Thus, the production process of the present invention enables full realization of the production of the peptide of interest on an industrial scale.

One object of the present invention on the production of GLP-1(7-36)NH₂ is to relieve aggregation and increase solubility at the time of reaction of modification enzymes such as an amidation enzyme described above through a peptide comprising GLP-1(7-37) having a helper peptide added thereto. In order to further investigate the usefulness of the present invention, RHHGP[G], RHHGP-1, GLP-1(7-37), and GLP-1(7-36)NH₂ were purified and the pH dependency of the solubility of each peptide was examined. As a result, GLP-1(7-37) was shown to have a low solubility in the range of pH 5.0 to pH 7.0 as expected. On the other hand, RHHGP[G] had a high solubility in the range of from pH 4.0 to about pH 6.0. The result confirmed that the substitution of RHHGP[G] having a helper peptide for GLP-1(7-37) as an enzyme substrate is useful since the amidation enzyme reaction is carried out in the weak acid region.

In the experiment that investigated the pH dependency of solubility of each peptide, RHHGP[G] and RHHGP-1 have shown a sudden reduction in solubility at about pH 6.0 and about pH 6.4, respectively. GLP-1(7-36)NH₂ formed precipitates or microcystals with time. Thus, it is estimated that substances capable of increasing the solubility of RHHGP[G] and RHHGP-1 in the neutral to weak alkali region and substances capable of increasing the solubility of the peptide of interest in the weak acid to weak alkali region would be very useful in the production process.

Thus, after an intensive study to search such substances, the inventors of the present invention have found that aggregation can be effectively prevented by adding to the reaction mixture a surfactant (for example, the addition of 0.1% Tween 80) in the case of RHHGP[G] and RHHGP-1, a surfactant (for example, the addition of 0.3% or greater of Tween 80) and/or a salt (for example, 100 mM or greater of NaCl) in the case of GLP-1(7-36)NH₂, and could prove the usefulness thereof by introducing it to the production method of the present invention.

For the production process of GLP-1(7-36)NH₂ using the production method of the present invention, the yield of each step obtained in the purification of GLP-1(7-36)NH₂ on a 10 g scale is collectively shown in Table 1 (20 liters of the producing organism W3110/pGP97ompPR was cultured and an 8 liter portion of the culture broth was used for the purification).

**Table 1**

| Summary of production steps of GLP-1(7-36)NH₂ | | | |
|---|---|---|---|
| Steps | Amount of GLP-1 (7-37) / GLP-1(7-36)NH₂ | Yield of Unit process | Overall recovery |
| | (g) | (%) | (%) |
| Culture (corresponding to 8 L culture broth) | 26.87 | 100 | 100 |
| OmpT reaction | 22.95 | 85.4 | 85.4 |
| SP sepharose chromato. | 20.22 | 98.8 | 76.2 |
| Amidation enzyme reaction | 20.70 | 100 | 77.0 |
| Kex2 enzyme reaction | 18.70 | 90.4 | 69.6 |
| MacroPrep HS chromato. | 16.78 | 93.7 | 62.4 |
| Poros R2 chromato. | 13.48 | 80.4 | 50.2 |

The amount of GLP-1(7-37) is shown for the process from the culturing to the amidation enzyme reaction, and the amount of GLP-1(7-36)NH₂ is shown for the process from the Kex2 step to the Poros R2 chromatography step. The amount of GLP-1(7-37) / GLP-1(7-36)NH₂ was calculated from the ratio of the peak area of HPLC to the number of amino acids.

As is evident from Table 1, the yield of a unit process in each step is very high, and the overall yield was shown to be as very high as about 50%. Thus, it is clear that the process for producing peptides of the present invention can be applied to the production of GLP-1(7-36)NH₂ and that it can be scaled up to an industrial production level. Furthermore, since the yield of the unit process in the process of cleavage treatment reaction with OmpT protease and Kex2 protease is 85% and 90.4%, respectively, it was confirmed that each cleavage site region designed had amino acid sequences very suitable for the cleavage treatment reaction with the enzyme.

As hereinabove described, it was shown, as an example of the process for producing a GLP-1 derivative having an insulinotropic activity, that the present invention can relieve the problems in production processes due to the intrinsic physicochemical properties of the peptide of interest. The problems on production due to the physicochemical properties of the GLP-1 derivatives represented by GLP-1(7-37) and GLP-1(7-36)NH₂ that were specifically described can be overcome using the production method according to the present invention, and, needless to say, the present invention is also useful for the production of GLP-1 derivatives.

The above-mentioned production of GLP-1 derivatives can be effected by a method using a fusion protein having a protective peptide added thereto. However, when the peptide of interest is obtained in accordance with the invention from said fusion protein through the peptide of interest having a helper peptide added thereto, multiple cleavage site regions are provided, and therefore a multi-step method of cleaving the fusion protein will be required.

Thus, the inventors have investigated on methods for chemically or enzymatically cleaving each cleavage site region, and have confirmed that it is possible by a method other than the one that employs the cleavage with E. coli OmpT protease confirmed in particular in an Example described below. Representative examples of the site-specific cleavage method of peptide at such cleavage site regions include:
(1) a method in which the absence of cysteine residues in the amino acid sequences of the protective peptide and the peptide of interest is utilized, and cysteine is introduced at the C-terminal of the protective peptide, which is then cyanized, subjected to an alkali treatment to specifically cleave the fusion protein at said N-terminal of the cysteine;
(2) a method in which cleavage at each cleavage site region is carried out with Kex2 protease, but by using different amino acid sequences at the enzyme recognition sites, the reaction condition for one cleavage site region is designed so as not to permit cleavage at another cleavage site region; and
(3) a method in which cleavage at each cleavage site region is carried out with Kex2 protease, but by modifying amino acids in one cleavage site region (cleavage site region 2), the reaction condition for cleavage of the other cleavage site region (cleavage site region 1) is designed so as not to cause cleavage at the former cleavage site (cleavage site region 2), and after cleavage at the latter cleavage site region (cleavage site region 1), the peptide comprising a helper peptide and the peptide of interest is purified, and then the modified amino acid is modified again so that the former cleavage site region (cleavage site region 2) can be made possible to be cleaved with Kex2 protease.

### EXAMPLES

With a GLP-1 derivative as the peptide of interest, the present invention will now be explained in further detail with reference to the following Examples.

As a production process when the peptide of interest is GLP-1(7-37), as described above, the peptide can be produced by expressing a fusion protein comprising the protective peptide and GLP-1(7-37) in a conventional method, and then by excising GLP-1(7-37) directly from said protein. However, this method had problems on the purification process in which the gelling or aggregation of GLP-1(7-37) occurred during purification due to its physicochemical properties, which resulted in such problems as extreme reduction in recovery and incapacitation of regeneration of the resin. This drawback could be circumvented by changing the physicochemical properties of GLP-1(7-37) by adding a helper peptide thereto. The method for producing said peptide is now explained more specifically below.

### Example 1. Construction of plasmid

An expression plasmid pGP97ompPR that encodes a fusion protein (GP97ompPR) designed for the production of GLP-1(7-37) was prepared through the 4 steps shown below. The restriction enzyme treatment, the ligation reaction, the phosphorylation of the 5'-end, and PCR conditions were carried out according to conventional methods.

### (1) Step 1 Construction of pG117S4HR6GLP-1

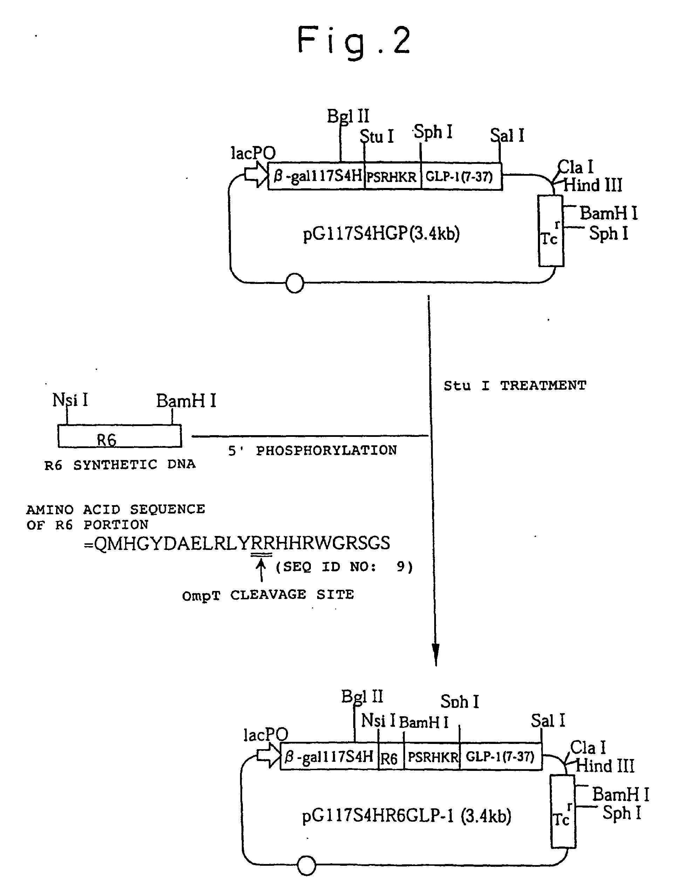

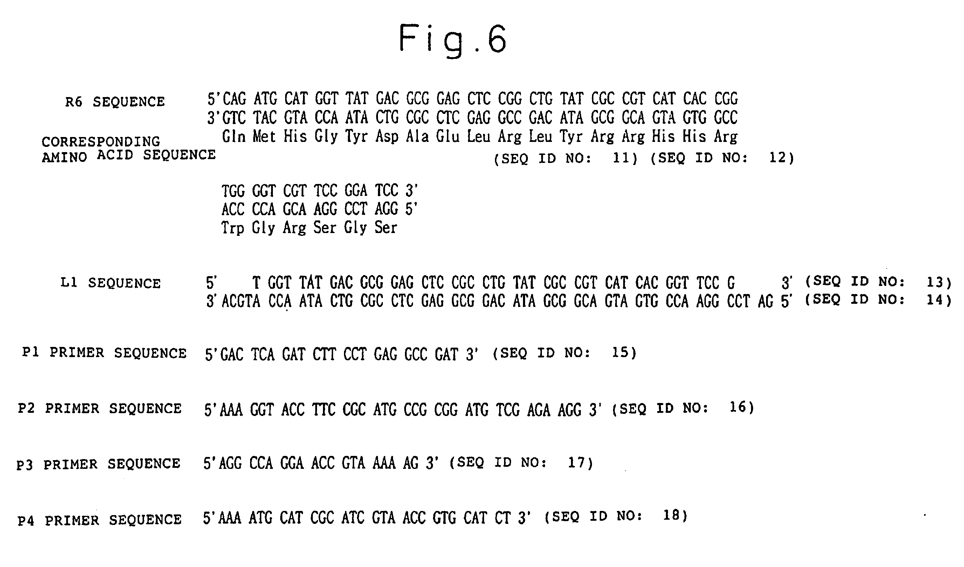

In order to design GP97ompPR that can be cleaved with E. coli OmpT protease, R6 synthetic DNA (see Figure 6) encoding the amino acid sequence R6 (see Figure 2) having the Arg-Arg sequence, a recognition sequence for OmpT protease, was inserted into a StuI site of pG117S4HGP (see Japanese Unexamined Patent Publication (Kokai) No. 9(1997)-296000) to construct pG117S4HR6GLP-1 (Figure 2).

### (2) Step 2 Construction of pGP117S4HompRHKR

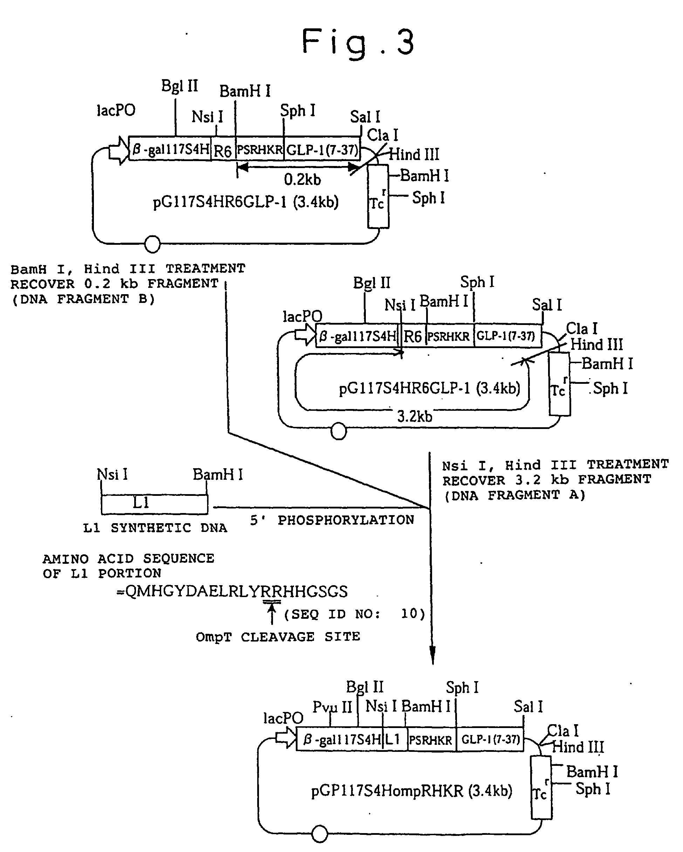

In order to further enhance the efficiency of cleavage by E. coli OmpT protease, the sequence of the R6 portion was changed. A 3.2 kb fragment (fragment A) obtained by cleaving pG117S4HR6GLP-1 with Nsi I and Hind III was ligated to a 0.2 kb fragment (fragment B) obtained by cleaving PG117S4HR6GLP-1 with BamH I and Hind III and L1 synthetic DNA (see Figure 6) encoding a portion of the amino acid sequence L1 (see Figure 3) having the Arg-Arg sequence, a recognition sequence for OmpT protease, to construct pGP117S4HompRHKR (Figure 3).

### (3) Step 3 Construction of pGP117S4HompRHPR

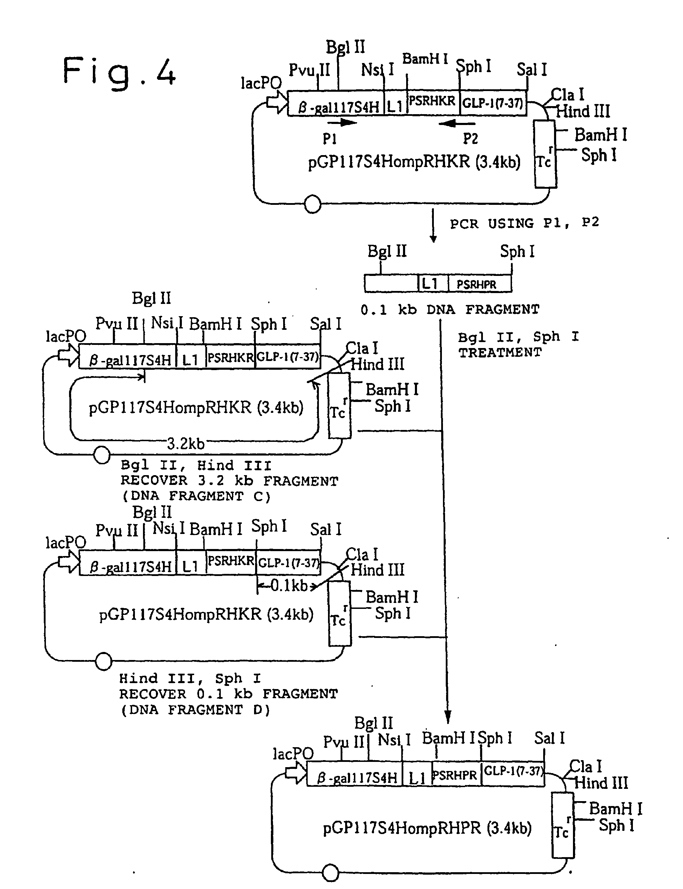

In order to make the E. coli OmpT protease recognition sequence and the Kex2 protease recognition sequence different from each other in the cleavage site region, Lys-Arg in the Kex2 protease recognition sequence was replaced with Pro-Arg. P1 primer and P2 primer (see Figure 6) were synthesized, which were subjected to PCR using pGP117S4HompRHKR as the template to construct a 0.1 kb DNA fragment. After treating the thus obtained DNA fragment with Bgl II and Sph I, it was ligated to a 3.2 kg fragment (fragment C) obtained by cleaving pGP117S4HompRHKR with Bgl II and Hind III and a 0.2 kb fragment (fragment D) obtained by cleaving pGP117S4HompRHKR with Sph I and Hind III to construct pGP117S4HompRHPR (Figure 4).

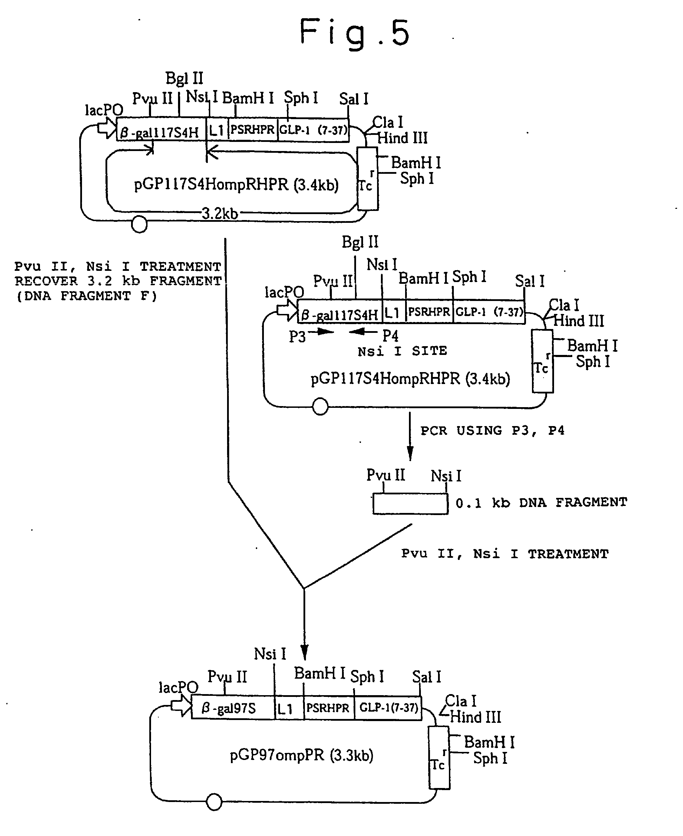

### (4) Step 4 Construction of pGP97ompPR (Figure 5)

In order to further reduce the size of the protective peptide portion, pGP97ompPR was constructed. P3 and P4 primers (see Figure 6) were synthesized, which were subjected to PCR using pGP117S4HompRHPR as the template to construct a DNA fragment. After treating the thus obtained DNA fragment with Pvu II and Nsi I, it was ligated to a 3.2 kb fragment obtained by cleaving pGP117S4HompRHPR with Pvu II and Nsi I to construct pGP97ompPR.

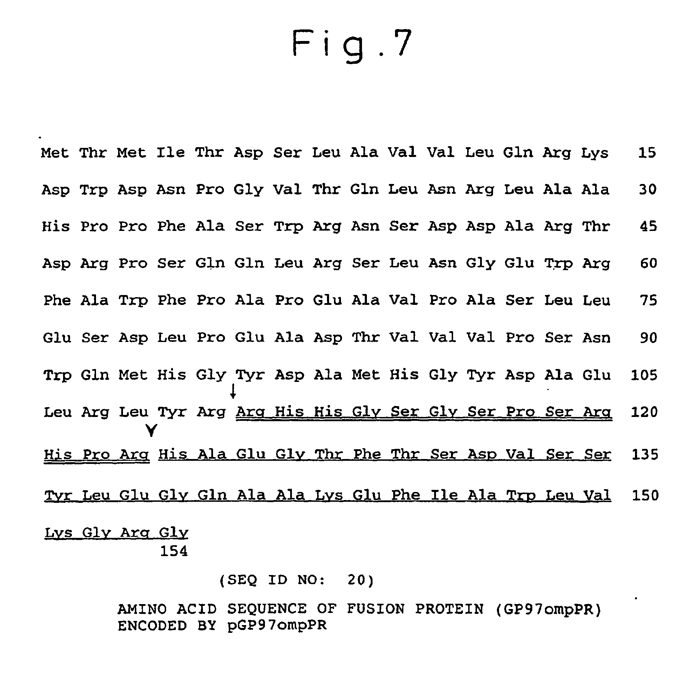

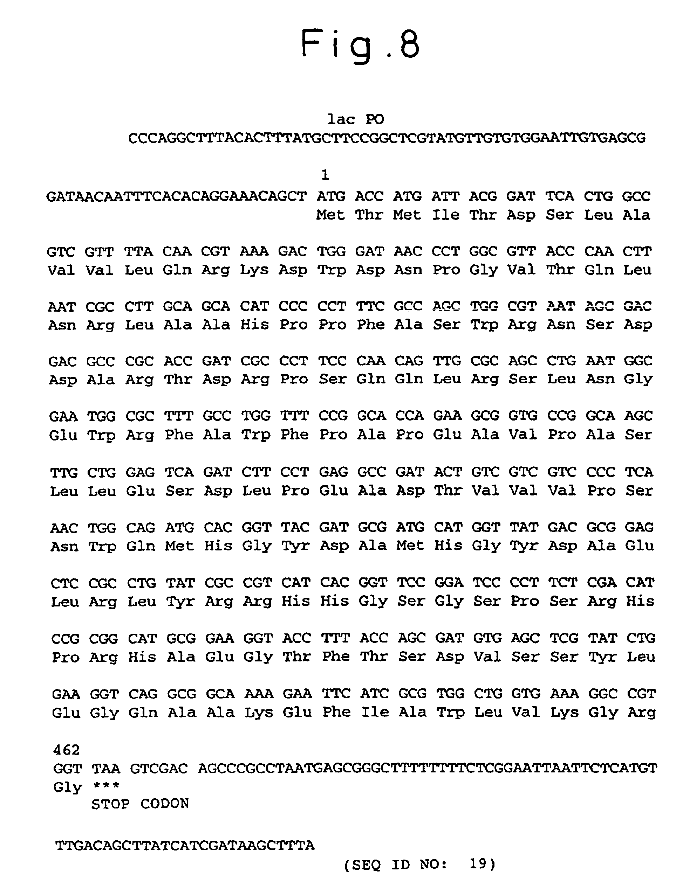

The amino acid sequence of the fusion protein (GP97ompPR) encoded by the thus constructed plasmid pGP97ompPR is shown in Figure 7, and the DNA nucleotide sequence encoding said amino acid sequence is shown in Figure 8.

### Example 2. Expression of fusion protein (GP97ompPR)

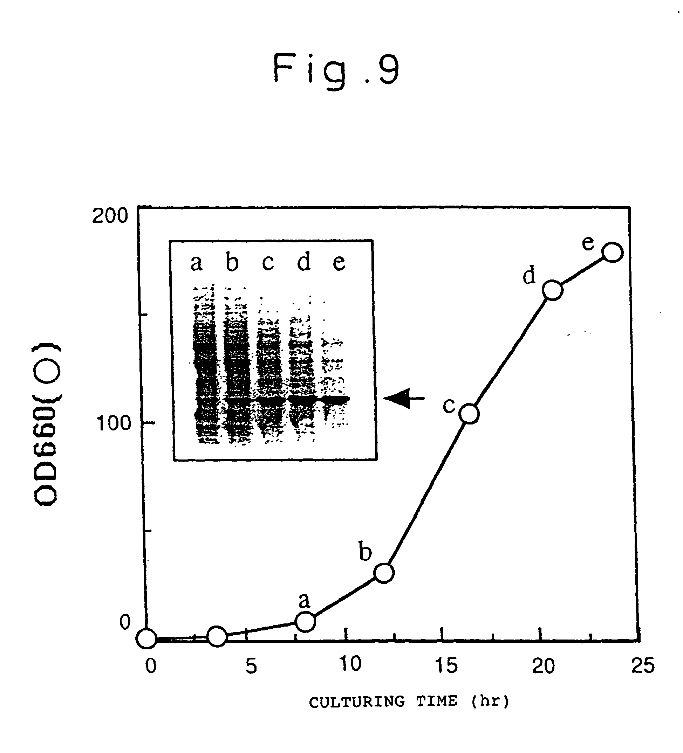

E. coli strain W3110 having pGP97ompPR was cultured in a culture medium (20 L, pH 7.0) containing 4 g/l K₂HPO₄, 4 g/l KH₂PO₄, 2.7 g/l Na₂HPO₄, 0.2 g/l NH₄Cl, 1.2 g/l (NH₄)₂SO₄, 4 g/l yeast extract, 2 g/l MgSO₄·7H₂O, 40 mg/l CaCl₂·2H₂O, 40 mg/l FeSO₄·7H₂O, 10 mg/l MnSO₄·nH₂O, 10 mg/l AlCl₃·6H₂O, 4 mg/l CoCl₂·6H₂O, 2 mg/l ZnSO₄·7H₂O, 2 mg/l Na₂MoO₄·2H₂O, 1 mg/l CuCl₂·2H₂O, 0.5 mg/l H₃BO₄, and 10 mg/l tetracycline in a 30-liter jar fermenter at a temperature of 32°C for 12 hours and thereafter at 37°C for 24 hours while adding glucose. The pH of the medium was controlled at pH 7.0 by adding a 28% ammonium solution. Figure 9 is the result of changes in cell density (OD660), and the expression of the fusion protein (GP97ompPR) at the time of sampling that were analyzed with 16% SDS-PAGE. GP97ompPR was expressed as the inclusion body and accounted for 30% or more of the total cellular protein at the end of culturing.

After culturing, the culture broth was homogenized at 500 Kg/cm2 using a Manton-Gaulin homogenizer (15M-8TA), and then was centrifuged to collect the precipitate fraction (inclusion body). Then, in order to wash the obtained precipitate, an equal amount of deionized water was added to the culture broth. After suspension, centrifugation was repeated to collect the precipitate. The washing process was repeated again and finally the obtained precipitate was suspended in a suitable amount of deionized water.

### Example 3. Processing of GP97ompPR with E. coli OmpT protease

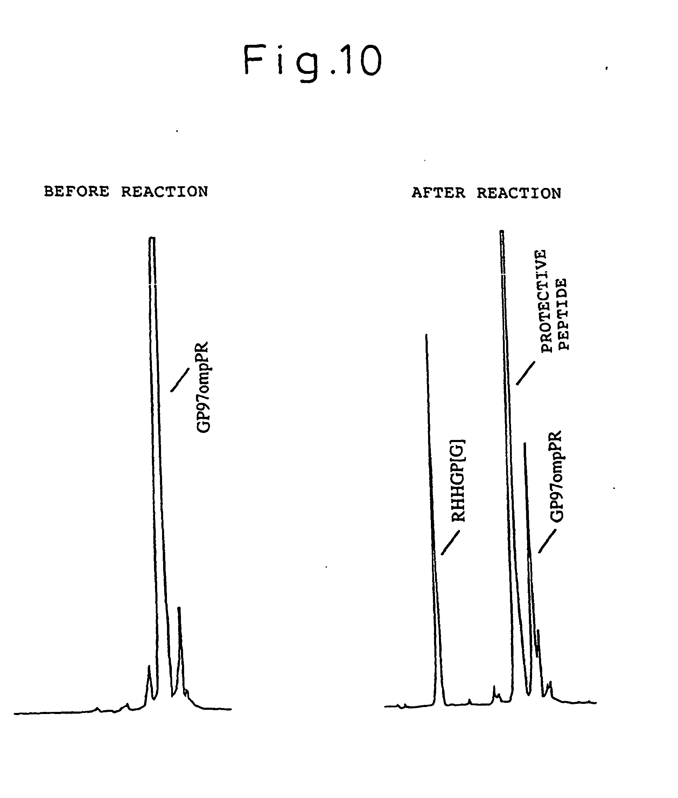

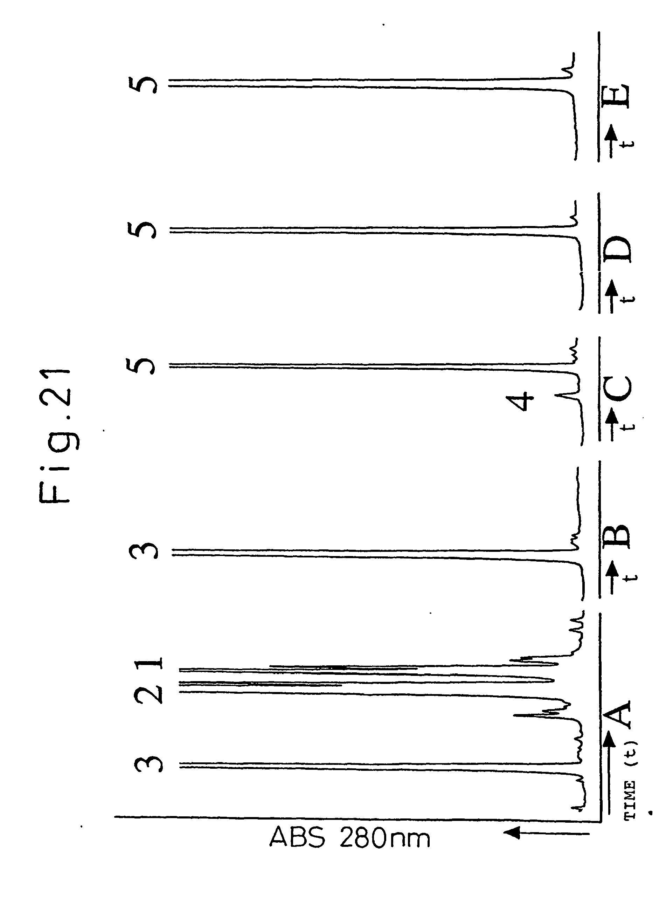

The obtained suspension of the inclusion body was diluted to give an OD660 value of 1000. From the liquid a 1000 ml portion was taken, to which 250 ml of 1M Tris-HCl for which pH has not been adjusted, 10 ml of 0.5 M EDTA (pH 8.0), and 1200 g of powdered urea was added, and then deionized water was added thereto to give a final volume of 5000 ml. Then HCl was used to adjust pH at 7.5 and the liquid was heated to 37 °C for 2 hours. This procedure initialed the action of E. coli OmpT protease present in the inclusion body, and GP97ompPR was cleaved and RHHGP[G] was released. Figure 10 is the result of the RHHGP[G] that was excised from GP97ompPR and then was analyzed with reverse phase HPLC. The analysis used a YMC PROTEIN-PR column, a 10% acetonitrile solution containing 0.1% trifluoroacetic acid as solution A and a 70% acetonitrile solution containing 0.095% trifluoroacetic acid as solution B, with a flow rate of 1 ml/min and a linear gradient of solution B from 44% to 70% in 13 minutes. This procedure cleaved 85% of GP97ompPR and at the end of the reaction a peak corresponding to RHHGP[G] was obtained (Figure 21 A).

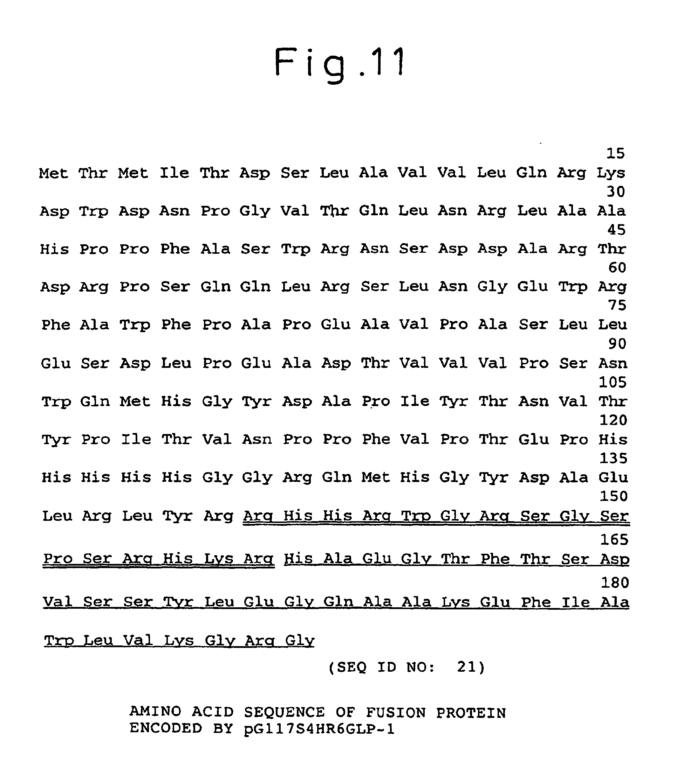

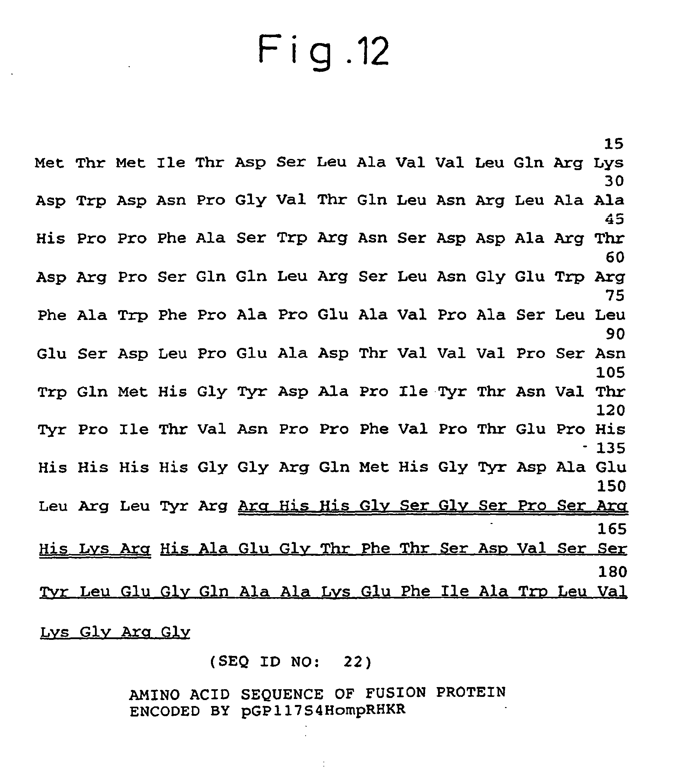

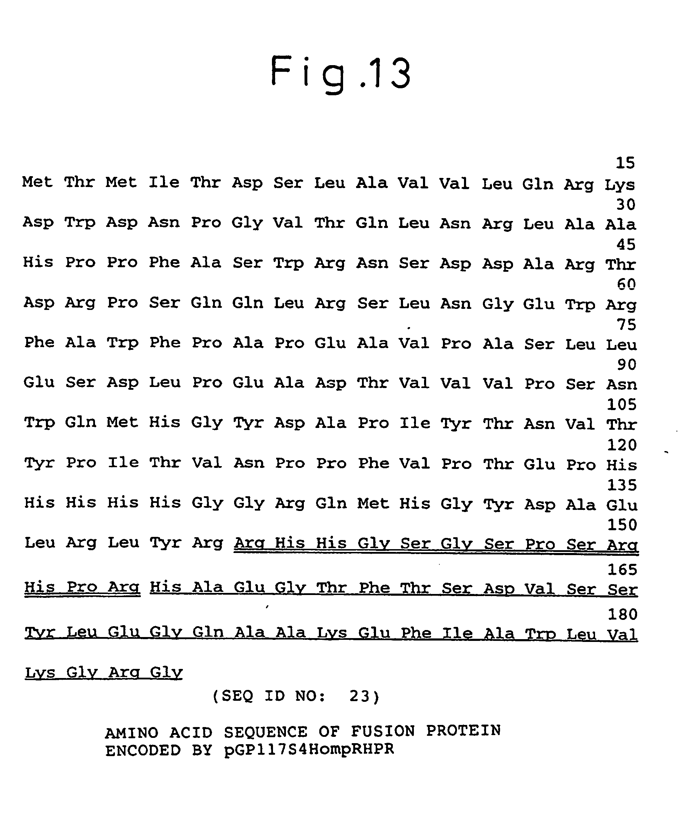

The excision of GLP-1(7-37) having a helper peptide added thereto from the fusion protein by the cleavage treatment with E. coli OmpT protease was not limited to the fusion protein derived from pGP97ompPR, and was also possible for fusion proteins derived from pG117S4HR6GLP-1, pGP117S4HompRHKR and pGP117S4HompRHPR constructed in Example 1. The amino acid sequences of these fusion proteins derived from plasmids are shown in Figures 11, 12, and 13.

### Example 4. Purification of RHHGP[G]

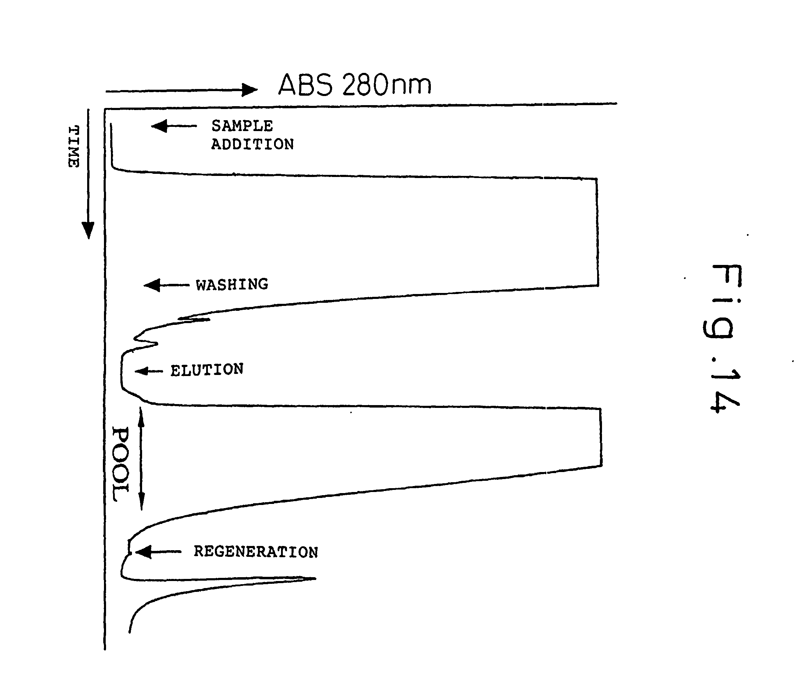

After the E. coli OmpT protease reaction, urea and Tween 80 were added to a concentration of 7M and 0.1%, respectively, and pH was adjusted to 8.0 with NaOH. The mixture was then centrifuged to obtain a supernatant. A column charged with SP-Sepharose BigBeads (Amersham Pharmacia Biotechnology) was equilibrated with 100 mM Tris HCl, pH 8.0, and then 20 mM Tris HCl, pH 8.0 / 5 M urea / 0.1% Tween 80. After the above supernatant was added to the equilibrated column, the column was washed in the same equilibration solution, and then was washed with 0.2 M NaCl / 20 mM Tris HCl, pH 8.0 / 0.1% Tween 80 and eluted with 0.5 M NaCl / 20 mM Tris HCl, pH 8.0 /0.1% Tween 80 (Figure 14).

The purity of RHHGP[G] in the eluate was as high as 98%. The reason why such a high purity peptide could be obtained by a simple procedure of the addition of a helper peptide followed by a stepwise elution from a low pressure chromatography column is that at the time of designing a helper peptide, the introduction of hydrophilic amino acids and isoelectric points were taken into account for purification with an ion exchange column.

Example 5 shows that the above result has greatly contributed to saving labor during the purification process after the present step.

### Example 5. Excision and purification of GLP-1(7-37) from RHHGP[G]

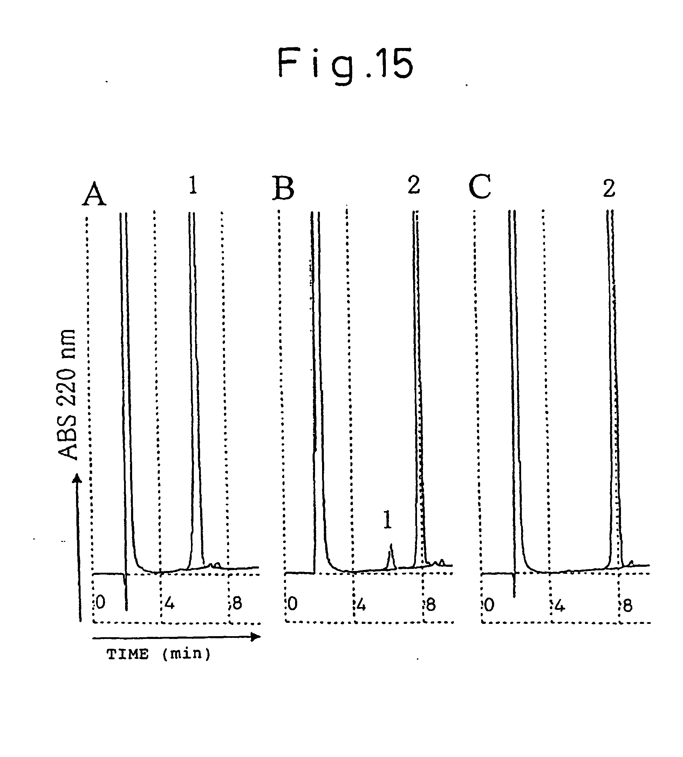

Processing with Kex2 protease was carried out using RHHGP[G], purified in Example 4, as the following reaction mixture: 5.0 mg/ml RHHGP[G], 20 mM Tris HCl, 0.1% Tween 80, 0.3 M NaCl, pH 8.0, 2.0 mM CaCl₂, and 8000 units/ml Kex2 protease (about 1.0 mg/L). A reaction rate of 95% was obtained in one hour (Figure 15-B). During the reaction, no precipitate formation of GLP-1(7-37) was observed.

Immediately after the enzyme reaction, ammonium acetate was added to a final concentration of 10 mM and was adjusted to pH 4.5 with HCl. To a Poros R2 column equilibrated with 10 mM ammonium acetate, pH 4.5, the above solution was loaded to a concentration of 10 mg GLP-1(7-37) equivalents / ml resin. After the column was washed in the same equilibration liquid and then in 0.2% acetic acid / 10% acetonitrile, and was eluted with 0.2% acetic acid / 40% acetonitrile. After removing acetonitrile, a lyophilized product was obtained. The recovery of GLP-1(7-37) was 80% with a purity of 99% (Figure 15-C).

Comparison of the method (see, for example, Japanese Unexamined Patent Publication (Kokai) 9(1997)-296000) for excising GLP-1(7-37) directly from a fusion protein comprising the protective peptide and the peptide of interest with the method of the present invention is shown in Table 2.

**Table 2**

| Comparison of GLP-1(7-37) production process | | | |
|---|---|---|---|
| A | | | |
| Process | Purity (%) | Yield of unit process (%) | Overall recovery (%) |
| Kex2 enzyme reaction | 15 | - | 100 |
| Acid precipitation treatment | - | 68 | 68 |
| Filtration | 75 | 88 | 60 |
| Cation exchange chromatography | 95 | 95 | 57 |
| Reverse phase chromatography | 99 | 72 | 41 |

| B | | | |
|---|---|---|---|
| Process | Purity (%) | Yield of unit process (%) | Overall recovery (%) |
| OmpT reaction | 24 | - | 100 |
| Filtration | 24 | 90 | 90 |
| Cation exchange chromatography | 99 | 99 | 89 |
| Kex2 enzyme reaction | 96 | 90 | 80 |
| Reverse phase chromatography | 98 | 80 | 64 |

A is based on a method of excising GLP-1(7-37) directly from a fusion protein comprising a protective peptide and the peptide of interest. B is based on a method of excising GLP-1(7-37) from a fusion protein having a protective peptide further added to the peptide of interest having a helper peptide added thereto.

By using RHHGP[G] as an intermediate, GLP-1(7-37) having a purity of 99% was obtained at ease and at a high yield. Since HPLC is not used in the purification of the present invention, it is, needless to say, easy to scale up to an industrial scale.

The following is an example wherein a peptide of interest requires modification. Since GLP-1(7-36)NH₂ is an amidated peptide, an amidation modification reaction is required.

### Example 6. An amidation modification reaction of GLP-1(7-37) having a helper peptide added thereto

RHHGP[G] obtained in Example 4 was converted to RHHGP-1 using an amidation enzyme. In order to determine the reaction condition for the case wherein RHHGP[G] was used as a substrate, optimization was carried out for pH, temperature, the concentration of copper sulfate, catalase concentration, substrate concentration, L-ascorbic acid concentration, and the concentration of the amidation enzyme in a reaction volume of 0.5 ml. Separation of RHHGP[G] and RHHGP-1 and analysis thereof were carried out using an ion exchange HPLC column (Poros S/H, Perceptive Biosystems) in the presence of 30 mM Britton-Robinson buffer (referred to hereinafter as the BR buffer) excluding barbital at a pH gradient elution (6.0 to 9.0).

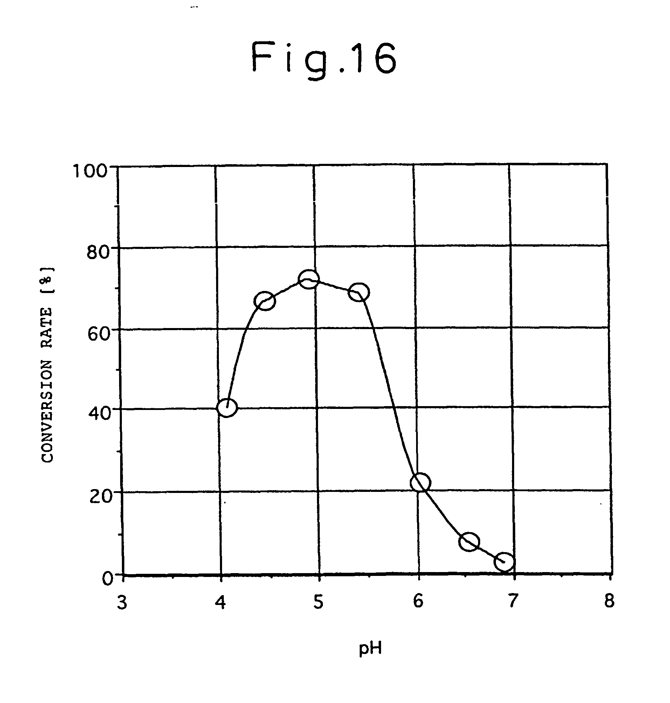

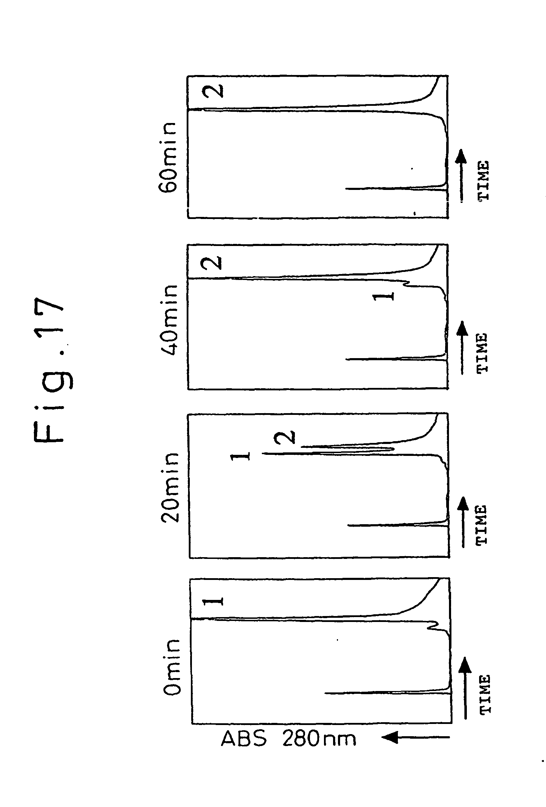

The optimum pH of the reaction condition was 5.0 to 5.5 (Figure 16). The optimum reaction condition was 10 mM sodium acetate, pH 5.0, 5.0 µM copper sulfate, 0.5 g/l L-ascorbic acid, 1 µM/ml catalase, 5.0 mg/ml RHHGP[G], a temperature of 32 °C, and 1500 units/ml amidation enzyme. To this condition was added Tween 80 (0.1%) that was found to have an aggregation suppression effect in Example 11 below, 5 liters of the RHHGP[G] solution was reacted in the above condition, and the reaction was stopped by adding EDTA. As a result of the reaction under the present condition, RHHGP[G] was converted with RHHGP-1 with a reaction rate of 98% or greater in one hour (Figure 17).

### Example 7. Excision of GLP-1(7-36)NH₂ from RHHGP-1 with Kex2 protease

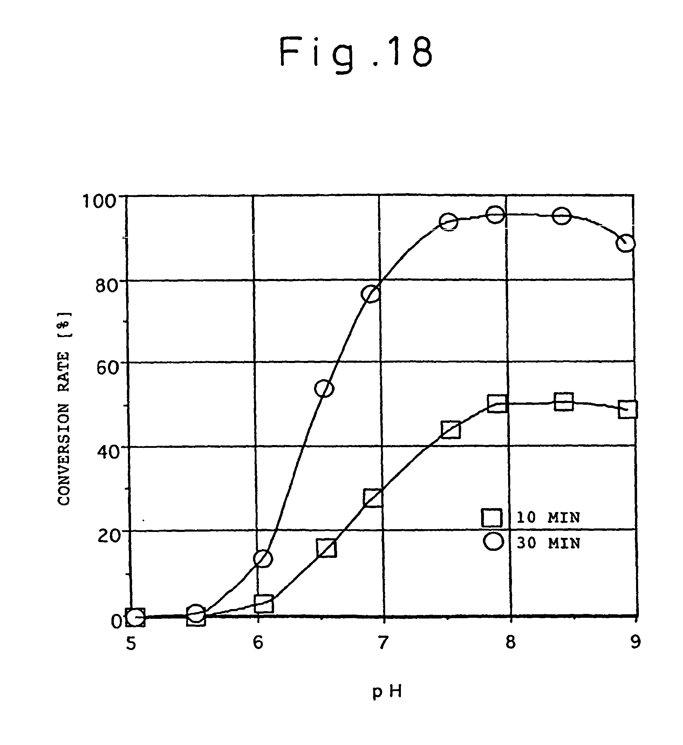

The processing reaction with Kex2 protease shows a pH dependency and activity change depending on the sequence of the portion that serves as the substrate (EP794255A). Accordingly, the optimization of pH, calcium chloride concentration and the amount of the enzyme added was carried out in a reaction volume of 0.5 ml. In the case of RHHGP-1, the reaction was shown to reach the maximum at pH 8.0 (Figure 18). The optimum reaction condition when RHHGP-1 was used as the substrate was set at 10 mM Tris-HCl, pH 8.0, 1 mM calcium chloride, 8,000 units/ml Kex2 protease and a reaction temperature of 30°C to 32°C. From the results in Example 11 below, aggregation could be avoided by setting the NaCl concentration in the reaction mixture at 0.1 M or greater and by further adding Tween 80 to a concentration of 0.1%.

By reacting the sample solution after the amidation reaction in Example 6 at 30°C under the present condition for 2 hours, a reaction rate of 95% or greater was obtained (Figure 21C). During the reaction, no precipitate formation of GLP-1(7-36)NH₂ was observed.

### Example 8. Purification of GLP-1(7-36)NH₂

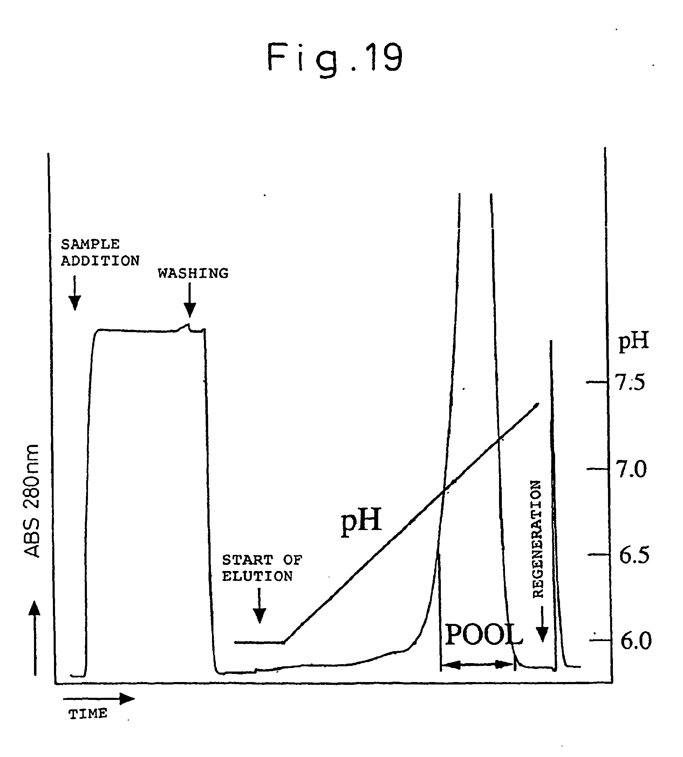

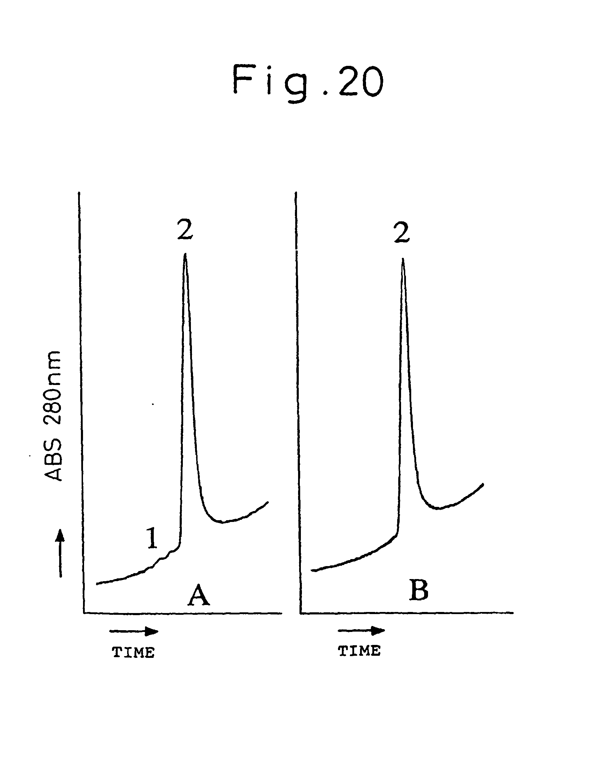

In order to remove impurities at minute amounts, a pH gradient elution using a cation exchange resin (MacroPrep High-S, BioRad) was used to separate GLP-1(7-36)NH₂ therefrom. The column was equilibrated with 20 mM BR buffer, pH 4.5 / 20 mM NaCl / 0.3% Tween 80. The composition of the sample solution was set at 0.3 M NaCl / 0.3% Tween 80, pH 4.5. The sample solution was added to the column, which was washed in the equilibration solution. Using the equilibration solution (solution A) and the elution solution (solution B; pH 7.0 with the same composition as the equilibration solution), GLP-1(7-36)NH₂ was eluted in a linear gradient from 50% solution B to 100% solution B (Figure 19) to make the proportion of the impurities less than 0.5% (Figure 20). In this process, most of the reagents added up to Example 7, unreacted products, and trace amounts of impurities were removed, and GLP-1(7-36)NH₂ with a purity of 98% or greater was obtained (Figure 21D). The pooled solution had a pH of 4.5 and was stored at 4 °C.

### Example 9. Final purification of GLP-1(7-36)NH₂

In the above Example 8, GLP-1(7-36)NH₂ with a purity of 98% or greater was obtained. When used as a pharmaceutical drug, however, not only the purity of the peptide of interest is important, but the contamination of non-peptide endotoxin must be avoided. Accordingly, in attempts to remove endotoxin etc., a preparative reverse phase HPLC column that is often used for the final purification of peptide drugs was used. But there were times when the aggregation and/or gelling of GLP-1(7-36)NH₂ occurred in the column. It was expected that the tendency of GLP-1(7-36)NH₂ to aggregate easily provides a major risk factor in scaling up.

On the other hand, although the hydrophobic chromatographic resin adsorbs substances utilizing the hydrophobicity thereof as with the reverse phase chromatographic resin, it is generally low in the density of functional groups and has an adsorption volume of 5 to 15 mg/ml resin. However, there are many kinds of carriers and many kinds of functional groups, and therefore it is possible to choose peptides that give a high recovery rate even when they have a tendency to easily aggregate like GLP-1(7-36)NH₂. After investigating various functional hydrophobic chromatographic resins, we have found that resins comprising hydrophilic carriers having butyl groups, isobutyl groups, hexyl groups or phenyl groups resins or polystyrene resins represented by Poros R2 are suitable. One example of such a resin, Poros R2 (Perceptive Systems) is shown below.

A maximum adsorption volume of the resin for GLP-1(7-36)NH₂ is about 12 mg/ml resin, which is higher than the other hydrophobic chromatographic resins, and which results in a higher concentration of GLP-1(7-36)NH₂ during elution, suggesting that the resin is suitable for lyophilization.

A 800 ml column was equilibrated with 10 mM ammonium acetate, pH 4.5, to which was added the sample solution of Example 8. After washing with the equilibration solution, the column was further washed with 0.2% acetic acid, and was eluted with 0.2% acetic acid / 40% acetonitrile. The recovery of GLP-1(7-36)NH₂ was 80% and the purity was 98% (Figure 21E). The product only contains volatile acids at a low concentration and therefore could be easily lyophilized after removal of acetonitrile. After reconstituting the lyophilized product, endotoxin was measured by the gelling method (Limulus ES-II test, Wako Pure Chemicals Co., Ltd.) and was found to be below the detection limit (0.03 µ/mg or lower). The fact that the column procedure described in the above method permits the purification of high yield and high purity GLP-1(7-36)NH₂ and enables elution by a solvent that can be lyophilized is, needless to say, industrially very useful.

### Example 10. Alleviation of aggregation using a helper peptide

One object of the production method of the present invention that employs a helper peptide is to alleviate the aggregation property that is a characteristics derived from the physicochemical properties of the peptide of interest, which has been a problem in the conventional production method, and therefore it is necessary to investigate the presence of the alleviation. In the present Example, therefore, it was investigated whether the aggregation property of a peptide comprising a helper peptide and GLP-1(7-37) and a peptide comprising a helper peptide and GLP-1(7-36)NH₂ has been improved compared to that of GLP-1(7-37) and GLP-1(7-36)NH₂.

First, RHHGP[G] was purified and the improvement of the aggregation property compared to that of GLP-1(7-37) was investigated and at the same time substances that suppress aggregation were researched. Each of peptide samples, i.e. a sample prepared by purifying with SP-sepharose BigBeads chromatography for RHHGP[G], a sample prepared by amidating with purified RHHGP[G] for RHHGP-1, a sample prepared by cleaving RHHGP[G] with Kex2 protease for GLP-1(7-37), and a sample prepared by cleaving RHHGP-1 with Kex2 protease for GLP-1(7-36)NH₂, was purified to a purity of 98% by eluting with an acetonitrile concentration gradient using the Poros R2 column, and then lyophilized to prepare each sample.

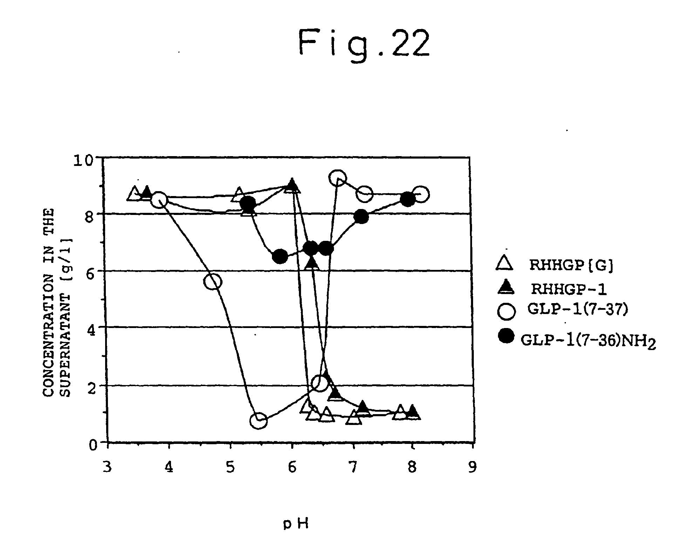

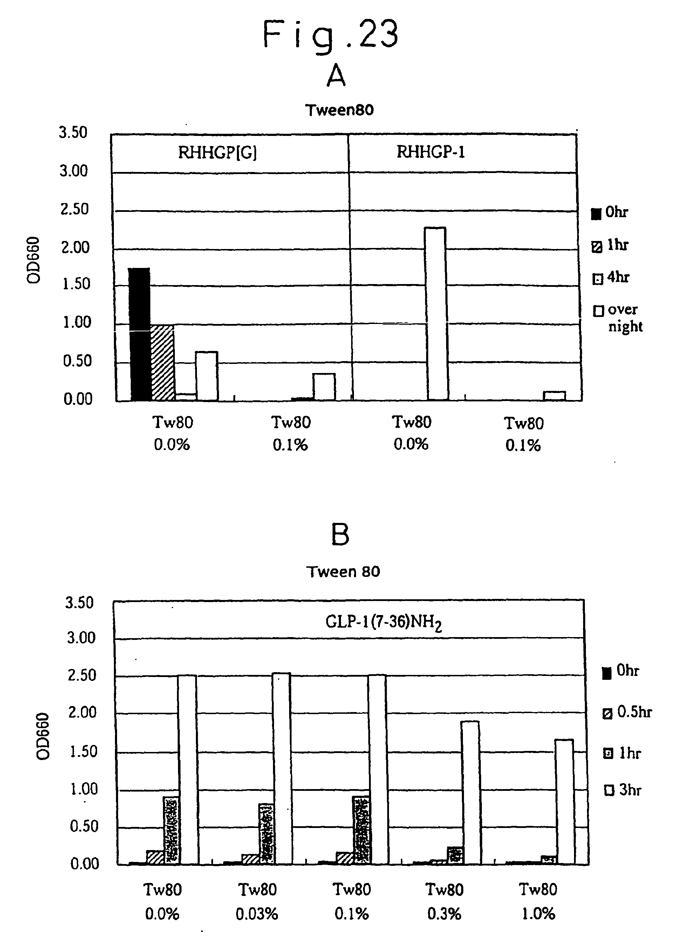

Since the aggregating property is considered to be associated with the solubility of each peptide, the pH dependency of the solubility of each peptide was investigated. The results are shown in Figure 22 and 23. It can be seen that GLP-1(7-37) is low in solubility in the range of pH 5.5 to pH 6.5, i.e., from the weak acid to the neutral condition which is optimum pH for the amidation enzyme reaction condition, indicating that it is inappropriate as a production intermediate.

On the other hand, although RHHGP[G] and RHHGP-1 start to show decreased solubility at about pH 6.2, they retain adequate solubility at least up to pH 5 or pH 6, and it was confirmed that they could be adequately efficiently reacted even under the amidation enzyme reaction condition.

### Example 11. Screening of substances having aggregation suppression

In the establishment of the production method of GLP-1(7-36)NH₂ on an industrial level using the production method of the present invention that employs a helper peptide, a substance that increases the solubility of RHHGP[G] and RHHGP-1 in the neutral to the weak alkali region and that maintain the solubility of GLP-1(7-36)NH₂ in the weak acid to the weak alkali region, if present, would be more preferred (GLP-1(7-36)NH₂ lags behind GLP-1(7-37) in the study shown in Figure 22, but it was found that it forms precipitates or microcrystals with time in the broad range of pH 5.3 to pH 8.0).

As the substances that are added to the solution so as to be able to maintain the solubility of each peptide, possible candidates include surfactants, sugars, salts, organic solvents and the like. Therefore, Tween 80 and Triton X100 as a surfactant, glucose, mannitol, and sucrose as a sugar, NaCl as a salt, and ethanol, glycerol, and DMSO as an organic solvent were tested for their ability of suppressing aggregation.

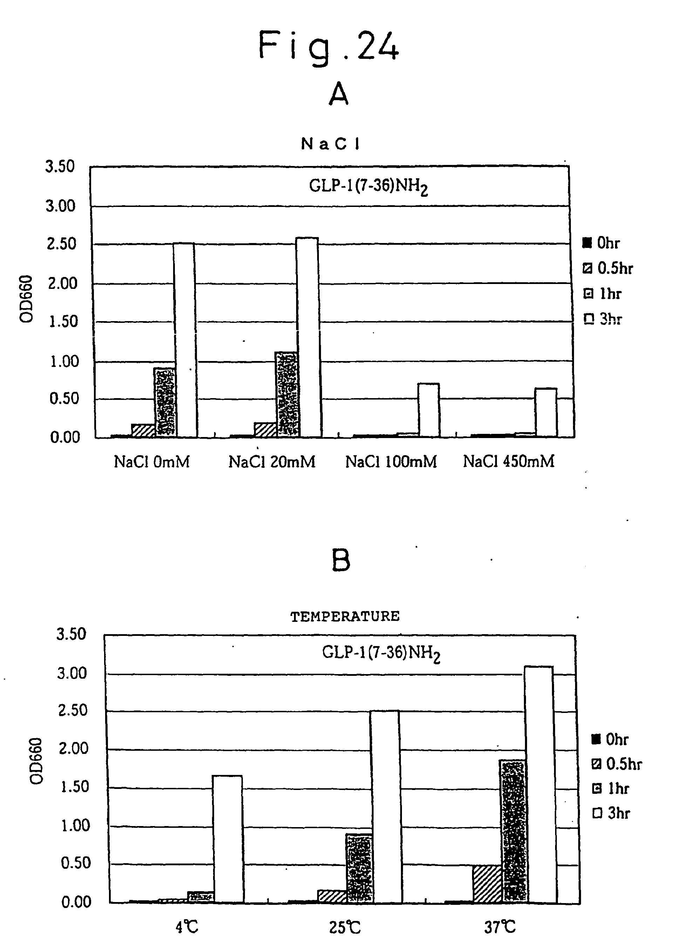

Ten mg/ml aqueous solutions of RHHGP[G], RHHGP-1, and GLP-1(7-36)NH₂ were prepared. To plastic tubes containing 0.1 ml of 300 mM BR buffer, pH 7.9, and 0.1 ml of 10-fold concentrated test sample solutions, 0.8 ml of each peptide solution was added, and pH was adjusted to levels that can easily cause aggregation, such as pH 7.5 to 8.5 for RHHGP[G], pH 8.0 for RHHGP-1, and pH 6.5 for GLP-1(7-36)NH₂, and then turbidity (absorbance at 660 nm) with time was monitored. Among the results obtained, the suppressive effects on aggregation by Tween 80, NaCl, and temperature are shown in Figure 23 and Figure 24.

It was found that precipitate formation was strongly suppressed with 0.1% Tween 80 in RHHGP[G] and RHHGP-1 (Figure 23A), but in GLP-1(7-36)NH₂ aggregation was suppressed by Tween 80 at 0.3% or higher (Figure 23B), NaCl at 100 mM or higher (Figure 24C), and/or al low temperature (Figure 24D).

From the above results, it was demonstrated that with regard to the pH condition in the actual production system, the addition of a salt and a surfactant are useful for suppressing the aggregation of the peptide of interest when the method for producing peptides of the present invention is used, and was indicated that the peptide of interest can be produced at high purity and high yields.

### Example 12. Methods of cleaving in the cleavage site region

Methods of cleaving used when cleaving is carried out at cleavage site regions were investigated. Thus, when the cleavage method for each of the case (1) cleavage site region 1: cyanization / alkalinization, cleavage site region 2: Kex2 protease; (2) cleavage site region 1: Kex2 protease, cleavage site region 2: Kex2 protease; and (3) cleavage site region 1: DTNB (5,5'-dithiobis-(2-nitrobenzoic acid)) addition / Kex2 protease, cleavage site region 2: reduction / Kex2 protease was investigated using the peptide of interest as GLP-1(7-37) and GLP-1(7-36)NH₂, it was found that cleavage by a multi-step cleavage method can be carried out by a chemical or enzymatic treatment when a fusion protein comprising a protective peptide is used in the production method of the present invention.

Results for each method are shown below. In order to enhance hydrophilicity of the polypeptide comprising a helper peptide and GLP-1(7-37) formed after cleavage and thereby to improve the solubility at neutral pH, 4 contiguous histidine sequences were introduced in all helper peptides.

### A. A method of producing GLP-1(7-37) through specific cleavage at the cysteine residue using method (1)

This method chemically cleaves a cysteine residue in one cleavage site region (cleavage site region 1) when the peptide of interest does not contain cysteine. For example, it was confirmed that after the fusion protein was expressed and obtained and a cysteine residue was cyanized with CADP (1-cyano-4-dimethylamino-pyridinium tetrafluoroborate), it could be specifically cleaved at the N-terminal side of cysteine of the cleavage site region by an alkali (NaOH) treatment.

Thus, PGGRPSRHKR (SEQ ID NO: 6) was selected as an amino acid sequence in cleavage site region 1 adjacent to the amino acid sequence of GCHHHH (SEQ ID NO: 5) as a helper peptide, and was introduced into a fusion protein. Said fusion protein was dissolved at 30 mg/ml in 50 mM Tris-HCl, pH 8.2, 5 M urea, and 10 mM DTT (dithiothreitol), and incubated in a 30°C incubator for 30 minutes to completely reduce cysteine. This was subjected to a gel-filtration column (manufactured by Pharmacia, PD10 column) that had been equilibrated with 10 mM Tris-HCl, pH 8.2, and 5 M urea to remove DTT.

In order to cyanize cysteine, acetic acid was added to a final concentration of 0.1 M, and CADP at 4-fold mole greater amounts than cysteine was added thereto and was reacted at 30°C for 1 hour. Cyanization reaction was confirmed by determining the residual SH groups with DTNB.

The above fusion protein was specifically cleaved at the cyanized cysteine site by removing an excess of reagents with a PD10 column equilibrated with 10 mM acetic acid and 5 M urea, adding NaOH to a final concentration of 50 mM, and then leaving it at room temperature for 30 minutes. The rate of cleavage was about 50 to 70%.

The other cleavage site region (cleavage site region 2) was subjected to a cleavage treatment in a similar manner to Example 7.

### B. Cleavage of a cleavage site region with Kex2 protease using method (2)

It was designed that while each cleavage site region has a Kex2 protease cleavage site region, the amino acid sequence of one cleavage site region (cleavage site region 2) is hardly cleaved under the reaction condition for the cleavage of the other cleavage site region (cleavage site region 1).

It is known that in the optimization of a cleavage recognition sequence in the cleavage site region of Kex2 protease, the charge of the amino acid residue at the P4 subsite, in addition to the P1 and P2 subsites, greatly influences the activity of the enzyme and that the fusion protein is not cleaved at all under a certain condition especially when an acidic amino acid is present at the P4 subsite (Japanese Unexamined Patent Publication (Kokai) 9(1997)-296000). By utilizing this, for example, by introducing aspartic acid (D) into the P4 subsite as the amino acid sequence SDHKR (SEQ ID NO: 8) of cleavage site region 2 adjacent to an amino acid sequence comprising HRHKRSHHHH (SEQ ID NO: 7) as a helper peptide, 90% or greater was cleaved with Kex2 protease in the cleavage of cleavage site region 1 in the fusion protein but cleavage site region 2 was protected from the cleavage. The result revealed that cleavage site region 1 can be specifically cleaved by introducing aspartic acid into the P4 subsite.

In an ion exchange chromatography and a column chromatography (for example, Cellulofine C-200 column chromatography) having a gel filtration ability that were carried out in order to isolate the obtained GLP-1(7-37) having a helper peptide added thereto, the desired GLP-1(7-37) having a helper peptide added thereto was specifically adsorbed. Some of the unreacted fusion protein also adsorbed but they were readily separated by a salt concentration gradient. The recovery was 94%. The chromatography can also be applied to GLP-1(7-36)NH₂ having another helper peptide added thereto.

Subsequently, after an amidation modification reaction, Kex2 protease cleavage treatment was carried out at cleavage site region 2 in order to excise GLP-1(7-36)NH₂ from GLP-1(7-36)NH₂ having a helper peptide added thereto. It was found that under the first cleavage condition which contained urea (denaturant) and which was alkaline, Kex2 protease did not cleave cleavage site region 2, but in the absence of urea cleavage site region 2 could be recognized by choosing an appropriate pH. It was shown that the optimum pH was 6.5 to 7.3 and thus cleavage was not easy at pH 8.2 which is a cleavage reaction condition in cleavage site region 1.

This is because the above GLP-1(7-36)NH₂ having a helper peptide added thereto is different from the fusion protein having a protective peptide further added thereto and is soluble in a wide pH range even in the absence of urea. Thus, Kex2 protease was allowed to react and the peptide was separated and determined with a reverse phase HPLC with the cleavage of 98% or greater being defined as complete. Since the system does not contain urea, the activity loss of Kex2 protease hardly occurs. Thus, it has an advantage that the amount of Kex2 protease required is extremely small, with a molar ratio to the substrate being 1:20000 to 1:40000.

As mentioned above, it was demonstrated that GLP-1(7-37) and GLP-1(7-36)NH₂ could be produced by using the same enzyme in cleavage treatment of each cleavage site region.

### C. Cleavage method utilizing a specific modification of cysteine using method (3)

This method is almost similar to method (2) and, when the peptide of interest does not contain cysteine residues, it modifies the cysteine residue introduced into the P4 subsite of one cleavage site region (cleavage site region 2), i.e. it protects the cleavage site region 2 from cleavage at the time of Kex2 protease cleavage reaction at the other cleavage site region (cleavage site region 1) by treating the side chain of cysteine with DTNB (dithionitriobenzoic acid), carries out reduction after a peptide comprising GLP-1(7-37) having a helper peptide added thereto was obtained, to thereby cleave cleavage site region 2 with Kex2 protease. Representative examples of such modification reactions include sulfonation and asymmetric disufidization with DTNB, but are not limited to these reactions as long as the method gives a negative charge to the cysteine side chain. For example, the amino acid sequence of cleavage site region 2 adjacent to the amino acid sequence comprising HRHKRSHHHH (SEQ ID NO: 7) as a helper peptide is introduced into a fusion protein as SCHKR (SEQ ID NO: 24).

The fusion protein designed as above was expressed, the fusion protein obtained was subjected to a DTNB treatment, and cysteine was made an asymmetric disulfide. After confirming the complete modification of cysteine, cleavage treatment reaction with Kex2 protease was carried out, and then it was confirmed that a peptide comprising GLP-1(7-37) having a helper peptide added thereto in an quantitative manner could be obtained. Thus, by changing the P4 subsite of the amino acid sequence recognition site of Kex2 protease at cleavage site region 2 to cysteine, and by introducing a negative charge in a side chain-specific manner, cleavage site region 2 was protected from cleavage at the time of cleavage with Kex2 protease at cleavage site region 1.

Then, purification and an amidation reaction was carried out as in the above B, DTT was added to reduce GLP-1(7-36)NH₂ having a helper peptide added thereto, hydrophobic column chromatography was carried out to purify to a purity of 98%, and then the product was lyophilized. When the product was dissolved at 5 mg/ml in 20 mM BisTris, pH 7.0, and 2 mM calcium chloride, 1000 units/ml of Kex2 protease was added thereto, and the mixture was reacted at 30°C, GLP-1(7-36)NH₂ having a helper peptide added thereto was specifically cleaved to give GLP-1(7-36)NH₂.

Although cleavage site region 2 was in a reduced state in this example, it is possible, needless to say, to modify it to have a positive charge and thereby to change its reactivity to Kex2 protease.

### Industrial Applicability

In accordance with the present invention, a method for producing biologically active peptides efficiently and at low cost on an industrial scale is provided. Specifically, it was demonstrated, as described in Examples of the present invention, that GLP-1 derivatives for which industrial production has been difficult can be produced at high purity and high yields. The production method of the present invention can be applied to the production of biologically active peptides other than GLP-1 derivatives and thus are industrially extremely useful.

## Claims

1. A process for producing a peptide having a desired biological activity, comprising the steps of:
(1) culturing cells transformed with an expression vector having a nucleotide sequence encoding a fusion protein, said fusion protein comprising a peptide of interest of not greater than 200 amino acids, a protective peptide which is heterologous to the peptide of interest and protects the peptide of interest from degradation by intracellular proteases, and a helper peptide, wherein said fusion protein has cleavage sites between the protective peptide, the helper peptide and the peptide of interest so that said fusion protein comprises two cleavage sites;
(2) harvesting said fusion protein from said culture;
(3) cleaving said fusion protein so as to liberate said protective peptide and thereby obtain said peptide of interest with said helper peptide attached;
(4) purifying said peptide of interest with said helper peptide attached;
(5) optionally subjecting said peptide of interest with said helper peptide attached to a modification reaction;
(6) cleaving said peptide of interest with said helper peptide attached so as to liberate said helper peptide and thereby obtain said peptide of interest; and
(7) purifying said peptide of interest;
wherein said helper peptide alters the physicochemical properties of the peptide of interest, in order to circumvent problems in the purification and/or modification of the peptide of interest that derive from the physicochemical properties of the peptide of interest.

2. The process according to claim 1, wherein the helper peptide alters the isoelectric point of the peptide of interest and/or increases the solubility of the peptide of interest in the purification and/or cleavage and/or modification step.

3. The process according to claim 2, wherein the fusion protein, the peptide of interest with the helper peptide attached, and the protective peptide have different isoelectric points.

4. The process according to any preceding claim, wherein said helper peptide has 5 to 50 amino acid residues.

5. The process according to any preceding claim, wherein said peptide of interest having a helper peptide added thereto has an isoelectric point of 8 to 12.

6. The process according to any preceding claim, wherein said protective peptide has 30 to 200 amino acid residues.

7. The process according to any preceding claim, wherein an ion exchange resin is used in the purification process.

8. The process according to claim 7, wherein said ion exchange resin is a cation exchange resin.

9. The process according to any preceding claim, wherein a reverse phase chromatography or a hydrophobic chromatography is used in the purification process.

10. The process according to any preceding claim, wherein a surfactant and/or a salt are added in at least one of steps (1) to (5) to maintain the solubility of the peptide of interest.

11. The process according to any preceding claim, wherein the host cell is a prokaryotic cell or a eukaryotic cell.

12. The process according to claim 11, wherein the host cell is Escherichia coli.

13. The process according to any of claims 1 to 12, wherein the isoelectric point of the peptide of interest having a helper peptide added thereto is 8 to 12.

14. The process according to any of claims 1 to 12, wherein the peptide of interest is an amidated peptide.

15. The process according to any of claims 1 to 12, wherein the peptide of interest is a GLP-1 derivative having an insulinotropic activity.

16. The process according to claim 15, wherein the GLP-1 derivative having an insulinotropic activity that has a helper peptide added thereto has an isoelectric point of 8 to 12.

17. The process according to claim 15 or 16, wherein the GLP-1 derivative having an insulinotropic activity has an isoelectric point of 4.5 to 9.0.

18. The process according to claim 15 or 16, wherein the GLP-1 derivative having a insulinotropic activity has an isoelectric point of 5.5 to 7.5.

19. The process according to any of claims 13 to 18, wherein an ion exchange resin is used in the purification process.

20. The process according to claim 19, wherein said ion exchange resin is a cation exchange resin.

21. The process according to any of claims 13 to 18, wherein a reverse phase chromatography or a hydrophobic chromatography is used in the purification process.

22. The process according to any of claims 13 to 18, wherein a surfactant and/or a salt is added to maintain the solubility of the peptide of interest.

23. The process according to any of claims 15 to 22, wherein the purity of the GLP-1 derivative obtained having an insulinotropic activity is 98% or greater.

24. The process according to any of claims 1 to 23, wherein the content of endotoxin in the final purified product is not greater than 0.03 units/mg.

25. The process according to any of claims 15 to 24 which further comprises the step of formulating the GLP-1 derivative having an insulinotropic activity as a pharmaceutical.

## Patentansprüche

1. Verfahren zur Herstellung eines Peptids mit einer gewünschten biologischen Aktivität, umfassend die Schritte:
(1) Kultivieren von Zellen, die mit einem Expressionsvektor mit einer Nukleotidsequenz, die ein Fusionsprotein codiert, transformiert worden sind, wobei das Fusionsprotein ein Peptid von Interesse umfaßt, das nicht größer ist als 200 Aminosäuren, ein Schutzpeptid, das heterolog zum Peptid von Interesse ist und das Peptid von Interesse vor Abbau durch intrazellulare Proteasen schützt, und ein Hilfspeptid, wobei das Fusionsprotein Schnittstellen zwischen dem Schutzpeptid, dem Hilfspeptid und dem Peptid von Interesse aufweist, so dass das Fusionsprotein zwei Schnittstellen umfasst;
(2) Ernten des Verschmelzungsproteins aus der Kultur;
(3) Abspalten des Verschmelzungsprotein, so dass das Schutzpeptid freigesetzt wird und **dadurch** das Peptid von Interesse mit dem daran gebundenen Hilfspeptid erhalten wird;
(4) Reinigen des Peptids von Interesse mit dem daran gebundenen Hilfspeptid;
(5) gegebenenfalls Unterziehen des Peptids von Interesse mit dem daran gebundenen Hilfspeptid einer Modifizierungsreaktion;
(6) Abspalten des Peptids von Interesse mit dem daran gebundenen Hilfspeptid, so dass das Hilfspeptid freigesetzt wird und **dadurch** das Peptid von Interesse erhalten wird; und
(7) Reinigen des Peptids von Interesse;
wobei das Hilfspeptid die physikochemischen Eigenschaften des Peptids von Interesse verändert, um Probleme bei der Reinigung und/oder Modifizierung des Peptids von Interesse zu umgehen, die sich aus den physikochemischen Eigenschaften des Peptids von Interesse ergeben.

2. Verfahren gemäß Anspruch 1, wobei das Hilfspeptid den isoelektrischen Punkt des Peptids von Interesse verändert und/oder die Löslichkeit des Peptids von Interesse im Reinigungs- und/oder Spaltungs- und/oder Modifizierungsschritt erhöht.

3. Verfahren gemäß Anspruch 2, wobei das Fusionsprotein, das Peptid von Interesse mit dem daran gebundenen Hilfspeptid und das Schutzpeptid unterschiedliche isoelektrische Punkte aufweisen.

4. Verfahren gemäß einem der vorstehenden Ansprüche, wobei das Hilfspeptid 5 bis 50 Aminosäurereste aufweist.

5. Verfahren gemäß einem der vorstehenden Ansprüche, wobei das Peptid von Interesse mit einem daran angefügten Hilfspeptid einen isoelektrischen Punkt von 8 bis 12 aufweist.

6. Verfahren gemäß einem der vorstehenden Ansprüche, wobei das Schutzpeptid 30 bis 200 Aminosäurereste aufweist.

7. Verfahren gemäß einem der vorstehenden Ansprüche, wobei im Reinigungsverfahren ein Ionenaustauscherharz verwendet wird.

8. Verfahren gemäß Anspruch 7, wobei das Ionenaustauscherharz ein Kationenaustauscherharz ist.

9. Verfahren gemäß einem der vorstehenden Ansprüche, wobei in dem Reinigungsverfahren eine Umkehrphasenchromatographie oder eine hydrophobe Chromatographie verwendet wird.

10. Verfahren gemäß einem der vorstehenden Ansprüche, wobei ein Tensid und/oder ein Salz in mindestens einem der Schritte (1) bis (5) zugesetzt wird, um die Löslichkeit des Peptids von Interesse aufrechtzuerhalten.

11. Verfahren gemäß einem der vorstehenden Ansprüche, wobei die Wirtszelle eine prokaryontische Zelle oder eine eukaryontische Zelle ist.

12. Verfahren gemäß Anspruch 11, wobei die Wirtszelle Escherichia coli ist.

13. Verfahren gemäß einem der Ansprüche 1 bis 12, wobei der isoelektrische Punkt des Peptids von Interesse mit einem daran angefügten Hilfspeptid 8 bis 12 ist.

14. Verfahren gemäß einem der Ansprüche 1 bis 12, wobei das Peptid von Interesse ein amidiertes Peptid ist.

15. Verfahren gemäß einem der Ansprüche 1 bis 12, wobei das Peptid von Interesse ein GLP-1-Derivat mit insulinotroper Aktivität ist.

16. Verfahren gemäß Anspruch 15, wobei das GLP-1-Derivat mit insulinotroper Aktivität, das ein daran angefügtes Hilfspeptid aufweist, einen isoelektrischen Punkt von 8 bis 12 aufweist.

17. Verfahren gemäß Anspruch 15 oder 16, wobei das GLP-1-Deriat mit insulinotroper Aktivität einen isoelektrischen Punkt von 4,5 bis 9,0 aufweist.

18. Verfahren gemäß Anspruch 15 oder 16, wobei das GLP-1-Derivat mit insulinotroper Aktivität einen isoelektrischen Punkt von 5,5 bis 7,5 aufweist.

19. Verfahren gemäß einem der Ansprüche 13 bis 18, wobei ein Ionenaustauscherharz in dem Reinigungsverfahren verwendet wird.

20. Verfahren gemäß Anspruch 19, wobei das Ionenaustauscherharz ein Kationenaustauscherharz ist.

21. Verfahren gemäß einem der Ansprüche 13 bis 18, wobei in dem Reinigungsverfahren eine Umkehrphasenchromatographie oder eine hydrophobe Chromatographie verwendet wird.

22. Verfahren gemäß einem der Ansprüche 13 bis 18, wobei ein Tensid und/oder ein Salz zugesetzt wird, um die Löslichkeit des Peptids von Interesse aufrechtzuerhalten.

23. Verfahren gemäß einem der Ansprüche 15 bis 22, wobei die Reinheit des erhaltenen GLP-1-Derivats mit insulinotroper Aktivität 98 % oder größer ist.

24. Verfahren gemäß einem der Ansprüche 1 bis 23, wobei der Gehalt an Endotoxin im gereinigten Endprodukt nicht größer als 0,03 Einheiten/mg ist.

25. Verfahren gemäß einem der Ansprüche 15 bis 24, das ferner den Schritt des Formulierens des GLP-1-Derivats mit insulinotroper Aktivität als Arzneimittel umfaßt.

## Revendications

1. Un procédé de production d'un peptide ayant une activité biologique souhaitée, qui comprend les étapes consistant à :
(1) cultiver les cellules ayant été transformées par un vecteur d'expression ayant une séquence de nucléotides codant pour une protéine de fusion, ladite protéine de fusion comprenant un peptide d'intérêt de pas plus de 200 acides aminés, un peptide protecteur qui est hétérologue au peptide d'intérêt et protège le peptide d'intérêt de la dégradation par les protéases intracellulaires, et un peptide auxiliaire, dans lequel ladite protéine de fusion a des sites de clivage entre le peptide protecteur, le peptide auxiliaire et le peptide d'intérêt de sorte que ladite protéine de fusion comprend deux sites de clivage ;
(2) récolter ladite protéine de fusion de ladite culture ;
(3) cliver ladite protéine de fusion de manière à libérer ledit peptide protecteur et ainsi obtenir ledit peptide d'intérêt avec ledit peptide auxiliaire lié ;
(4) purifier ledit peptide d'intérêt avec ledit peptide auxiliaire lié ;
(5) facultativement soumettre ledit peptide d'intérêt avec ledit peptide auxiliaire lié à une réaction de modification;
(6) cliver ledit peptide d'intérêt avec ledit peptide auxiliaire lié de manière à libérer ledit peptide auxiliaire et ainsi obtenir ledit peptide d'intérêt ; et
(7) purifier ledit peptide d'intérêt ;
dans lequel ledit peptide auxiliaire modifie les propriétés physico-chimiques du peptide d'intérêt de manière à pallier les problèmes dans la purification et/ou la modification du peptide d'intérêt qui proviennent des propriétés physico-chimique du peptide d'intérêt.

2. Le procédé selon la revendication 1, dans lequel le peptide auxiliaire modifie le point isoélectrique du peptide d'intérêt et/ou fait augmenter la solubilité du peptide d'intérêt dans l'étape de purification et/ou de clivage et/ou de modification.

3. Le procédé selon la revendication 2, dans lequel la protéine de fusion, le peptide d'intérêt avec le peptide auxiliaire lié, et le peptide protecteur ont différents points isoélectriques.

4. Le procédé selon l'une quelconque des revendications précédentes, dans lequel ledit peptide auxiliaire a de 5 à 50 résidus d'acides aminés.

5. Le procédé selon l'une quelconque des revendications précédentes, dans lequel ledit peptide d'intérêt ayant un peptide auxiliaire ajouté à celui-ci a un point isoélectrique de 8 à 12.

6. Le procédé selon l'une quelconque des revendications précédentes, dans lequel ledit peptide protecteur a de 30 à 200 résidus d'acides aminés.

7. Le procédé selon l'une quelconque des revendications précédentes, dans lequel une résine échangeuse d'ions est utilisée dans le procédé de purification.

8. Le procédé selon la revendication 7, dans lequel ladite résine échangeuse d'ions est une résine échangeuse de cations.

9. Le procédé selon l'une quelconque des revendications précédentes, dans lequel une chromatographie à polarité de phase inversée ou une chromatographie hydrophobe est utilisée dans le procédé de purification.

10. Le procédé selon l'une quelconque des revendications précédentes, dans lequel un surfactant et/ou un sel sont ajoutés dans au moins une des étapes (1) à (5) pour maintenir la solubilité du peptide d'intérêt.

11. Le procédé selon l'une quelconque des revendications précédentes, dans lequel la cellule hôte est une cellule procaryote ou une cellule eucaryote.

12. Le procédé selon la revendication 11, dans lequel la cellule hôte est *Escherichia coli.*

13. Le procédé selon l'une quelconque des revendications 1 à 12, dans lequel le point isoélectrique du peptide d'intérêt ayant un peptide auxiliaire ajouté à celui-ci est de 8 à 12.

14. Le procédé selon l'une quelconque des revendications 1 à 12, dans lequel le peptide d'intérêt est un peptide amidé.

15. Le procédé selon l'une quelconque des revendications 1 à 12, dans lequel le peptide d'intérêt est un dérivé de GLP-1 ayant une activité insulinotrope.

16. Le procédé selon la revendication 15, dans lequel le dérivé de GLP-1 ayant une activité insulinotrope qui a un peptide auxiliaire ajouté à celui-ci a un point isoélectrique de 8 à 12.

17. Le procédé selon la revendication 15 ou 16, dans lequel le dérivé de GLP-1 ayant une activité insulinotrope a un point isoélectrique de 4,5 à 9,0.

18. Le procédé selon la revendication 15 ou 16, dans lequel le dérivé de GLP-1 ayant une activité insulinotrope a un point isoélectrique de 5,5 à 7,5.

19. Le procédé selon l'une quelconque des revendications 13 à 18, dans lequel une résine échangeuse d'ions est utilisée dans le procédé de purification.

20. Le procédé selon la revendication 19, dans lequel ladite résine échangeuse d'ions est une résine échangeuse de cations.

21. Le procédé selon l'une quelconque des revendications 13 à 18, dans lequel une chromatographie à polarité de phase inversée ou une chromatographie hydrophobe est utilisée dans le procédé de purification.

22. Le procédé selon l'une quelconque des revendications 13 à 18, dans lequel un surfactant et/ou un sel est ajouté pour maintenir la solubilité du peptide d'intérêt.

23. Le procédé selon l'une quelconque des revendications 15 à 22, dans lequel une pureté du dérivé de GLP-1 obtenu ayant une activité insulinotrope est de 98% ou plus.

24. Le procédé selon l'une quelconque des revendications 1 à 23, dans lequel la teneur en endotoxine dans le produit final purifié n'est pas supérieure à 0,03 unité/mg.

25. Le procédé selon l'une quelconque des revendications 15 à 24, qui comprend en outre l'étape consistant à formuler le dérivé de GLP-1 ayant une activité insulinotrope en tant qu'un produit pharmaceutique.
